(19) Europäisches Patentamt / European Patent Office / Office européen des brevets

(11) EP 4 397 405 A1

(12) **EUROPEAN PATENT APPLICATION**
published in accordance with Art. 153(4) EPC

(43) Date of publication:
**10.07.2024 Bulletin 2024/28**

(21) Application number: **22864617.0**

(22) Date of filing: **31.08.2022**

(51) International Patent Classification (IPC):
***B01J 20/22*** *(2006.01)* ***C07D 239/26*** *(2006.01)* ***C07F 1/00*** *(2006.01)*

(52) Cooperative Patent Classification (CPC):
**B01J 20/22; C07D 239/26; C07F 1/00;** Y02C 20/40

(86) International application number:
**PCT/JP2022/032745**

(87) International publication number:
**WO 2023/033035 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2021 JP 2021141297**

(71) Applicants:
- **ENEOS Corporation
Chiyoda-ku
Tokyo 100-8162 (JP)**
- **Tokyo Institute of Technology
Tokyo 152-8550 (JP)**

(72) Inventors:
- **MATSUMOTO, Takaya
Tokyo 100-8162 (JP)**
- **KAWANO, Masaki
Tokyo 152-8550 (JP)**
- **OHTSU, Hiroyoshi
Tokyo 152-8550 (JP)**
- **USOV, Pavel
Tokyo 152-8550 (JP)**
- **WADA, Yuki
Tokyo 152-8550 (JP)**
- **SHIMADA, Terumasa
Tokyo 152-8550 (JP)**

(74) Representative: **Müller-Boré & Partner
Patentanwälte PartG mbB
Friedenheimer Brücke 21
80639 München (DE)**

# (54) CARBON DIOXIDE CAPTURING AGENT, METAL-ORGANIC FRAMEWORK, AND COMPOUND

(57) The invention provides a novel carbon dioxide adsorbent that can capture carbon dioxide and the like, the carbon dioxide adsorbent being a carbon dioxide adsorbent including a metal-organic framework, the metal-organic framework can capture and desorb carbon dioxide, an isolated voids are formed inside the metal-organic framework by the metal-organic framework's three-dimensional structure, the isolated voids are the space that can capture carbon dioxide and does not have a channel through which carbon dioxide can pass in an ordinary state, and although the three-dimensional structure of the framework changes during the process where carbon dioxide is captured within isolated voids and the process where carbon dioxide is released from isolated voids, the three-dimensional structure of the metal-organic framework when carbon dioxide is captured within the isolated voids is the same as when carbon dioxide is not captured within the isolated voids.

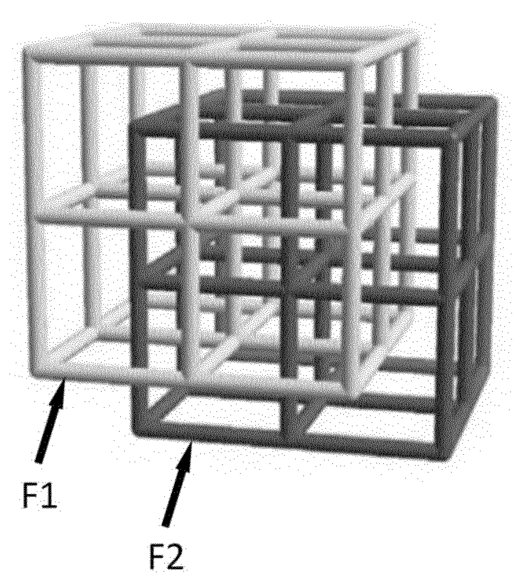


Fig. 1

EP 4 397 405 A1

## Description

BACKGROUND OF THE INVENTION

Technical Field

The present invention relates to a carbon dioxide adsorbent, a metal-organic framework that can be used for the carbon dioxide adsorbent, and a compound that can be used as a ligand for the metal-organic framework.

Background Art

In recent years, efforts toward a decarbonized society have created an urgent need to develop materials for capturing or storing carbon dioxide.

A porous network complex (PCN) composed of a combination of organic multidentate ligands and metal ions is attracting attention as a potential carbon dioxide- c because of its high design flexibility.

Many PCNs that exhibit carbon dioxide capture ability have been reported to date (see, for example, Non Patent Literature 1 and 2).

In addition, as a metal-organic framework (MOF) capable of trapping gas, a gate-opening-type MOF is known. However, in the gate-opening-type MOF, the volume after gas capture is larger than that before gas capture. In other words, in the gate-opening-type MOF, a structural change such as volumetric expansion occurs when gas is captured (see, for example, Non Patent Literature 3). When the structural change of the MOF is significant, the low durability of the MOF itself becomes a problem. Moreover, when the volumetric expansion of the MOF is significant, low durability is seen as a problem when the MOF is made into a molded body.

Citation List

Non Patent Literature

Non Patent Literature 1: Zhong Li et al, ACS Sustainable Chem. Eng. 8, 41, 15378-15404 (2020)
Non Patent Literature 2: Calogero Giancarlo Piscopo et al, ChemPlusChem 85, 538-547 (2020)
Non Patent Literature 3: Isotope News, August Issue, No.752, 12-15 (2017)


SUMMARY OF THE INVENTION

Technical Problem

An object of the present invention is to provide a novel carbon dioxide adsorbent that can capture carbon dioxide, a metal-organic framework that can be used for the carbon dioxide adsorbent, and a compound that can be used as a ligand for the metal-organic framework.

Solution to Problem

As a result of intensive studies to solve the problem described above, the present inventors found the solution to solve the problem. This has led to the completion of the present invention including the following subject matter of the invention.
In other words, the present invention encompasses the following.

[1] A carbon dioxide adsorbent comprising a metal-organic framework, wherein

the metal-organic framework can capture carbon dioxide and desorb carbon dioxide,
isolated voids are formed inside the metal-organic framework by the metal-organic framework's three-dimensional structure,
the isolated voids are the space that can capture carbon dioxide and does not have a channel through which carbon dioxide can pass in an ordinary state, and
although the three-dimensional structure of the framework changes during the process where carbon dioxide is captured within isolated voids and the process where carbon dioxide is released from isolated voids, the

three-dimensional structure of the metal-organic framework when carbon dioxide is captured within the isolated voids is the same as when carbon dioxide is not captured within the isolated voids .

[2] The carbon dioxide adsorbent according to [1], wherein the metal-organic framework has a BET-specific surface area of 1 $m^2/g$ or less in a specific surface area measurement using $N_2$.
[3] The carbon dioxide adsorbent according to [1] or [2], wherein the metal-organic framework forms an interpenetrating structure in which two frameworks interpenetrate each other.
[4] The carbon dioxide adsorbent according to any one of [1] to [3], wherein an element constituting the isolated voids and carbon dioxide captured within the isolated voids do not form a chemical bond in the metal-organic framework.
[5] The car carbon dioxide adsorbent according to any one of [1] to [4], wherein the metal-organic framework comprises at least one of groups II to XIV elements as a constituent element.
[6] The carbon dioxide adsorbent according to any one of [1] to [5], wherein the metal-organic framework comprises a halogen element as a constituent element.
[7] The carbon dioxide adsorbent according to any one of [1] to [6], wherein the metal-organic framework comprises a compound having a nitrogen-containing aromatic heterocycle as a ligand.
[8] The carbon dioxide adsorbent according to [7], wherein the compound having a nitrogen-containing aromatic heterocycle is a compound represented by the following Formula (1):

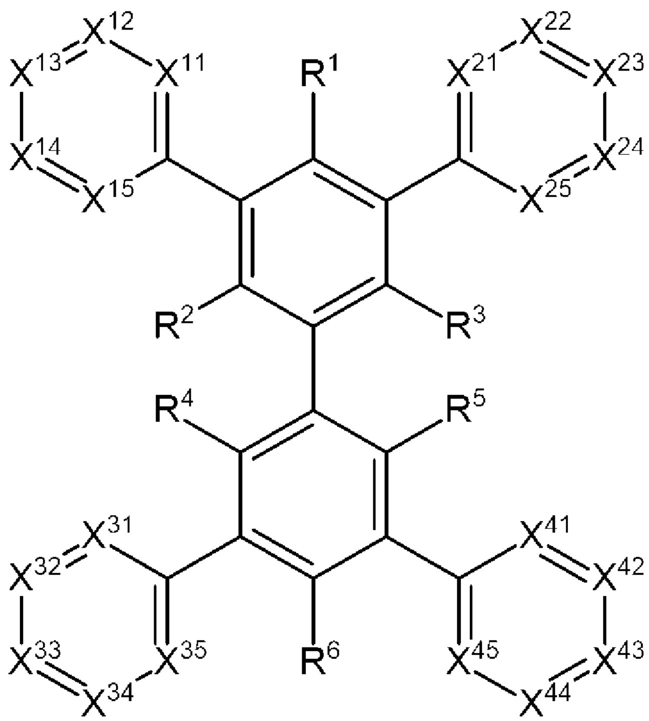

(1)

(in Formula (1),

$X^{11}$ and $X^{13}$ are N, and $X^{12}$, $X^{14}$, and $X^{15}$ are CR, or $X^{12}$ and $X^{14}$ are N, and $X^{11}$, $X^{13}$, and $X^{15}$ are CR, or $X^{11}$ and $X^{15}$ are N, and $X^{12}$, $X^{13}$, and $X^{14}$ are CR;
$X^{21}$ and $X^{23}$ are N, and $X^{22}$, $X^{24}$, and $X^{25}$ are CR, or $X^{22}$ and $X^{24}$ are N, and $X^{21}$, $X^{23}$, and $X^{25}$ are CR, or $X^{21}$ and $X^{25}$ are N, and $X^{22}$, $X^{23}$, and $X^{24}$ are CR;
$X^{31}$ and $X^{33}$ are N, and $X^{32}$, $X^{34}$, and $X^{35}$ are CR, or $X^{32}$ and $X^{34}$ are N, and $X^{31}$, $X^{33}$, and $X^{35}$ are CR, or $X^{31}$ and $X^{35}$ are N, and $X^{32}$, $X^{33}$, and $X^{34}$ are CR;
$X^{41}$ and $X^{43}$ are N, and $X^{42}$, $X^{44}$, and $X^{45}$ are CR, or $X^{42}$ and $X^{44}$ are N, and $X^{41}$, $X^{43}$, and $X^{45}$ are CR, or $X^{41}$ and $X^{45}$ are N, and $X^{42}$, $X^{43}$, and $X^{44}$ are CR;
each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and
$R^1$ to $R^6$ are each independently a hydrogen atom, a halogen atom, an alkyl group optionally substituted with

a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

[9] The carbon dioxide adsorbent according to [8], wherein the compound represented by Formula (1) is a compound represented by the following Formula (1-1):

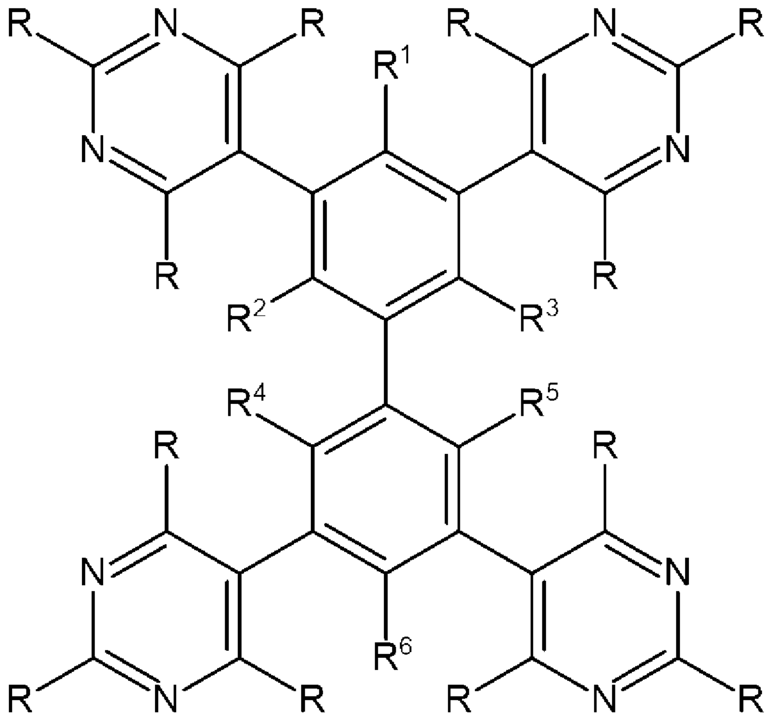

(1-1)

(in Formula (1-1),

each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and

$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

[10] The carbon dioxide adsorbent according to any one of [1] to [9], wherein the metal-organic framework has a composition formula, which is represented by $Cu_4I_4L$, with a ligand being L.

[11] A metal-organic framework comprising at least one of groups II to XIV elements as a constituent element and a compound represented by the following Formula (1) as a ligand:

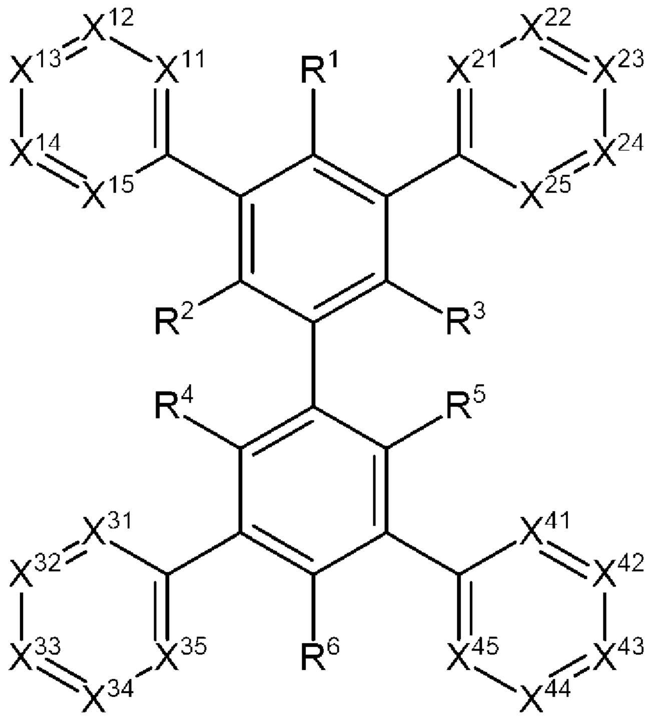

(1)

(in Formula (1),

$X^{11}$ and $X^{13}$ are N, and $X^{12}$, $X^{14}$, and $X^{15}$ are CR, or $X^{12}$ and $X^{14}$ are N, and $X^{11}$, $X^{13}$, and $X^{15}$ are CR, or $X^{11}$ and $X^{15}$ are N, and $X^{12}$, $X^{13}$, and $X^{14}$ are CR;
$X^{21}$ and $X^{23}$ are N, and $X^{22}$, $X^{24}$, and $X^{25}$ are CR, or $X^{22}$ and $X^{24}$ are N, and $X^{21}$, $X^{23}$, and $X^{25}$ are CR, or $X^{21}$ and $X^{25}$ are N, and $X^{22}$, $X^{23}$, and $X^{24}$ are CR;
$X^{31}$ and $X^{33}$ are N, and $X^{32}$, $X^{34}$, and $X^{35}$ are CR, or $X^{32}$ and $X^{34}$ are N, and $X^{31}$, $X^{33}$, and $X^{35}$ are CR, or $X^{31}$ and $X^{35}$ are N, and $X^{32}$, $X^{33}$, and $X^{34}$ are CR;
$X^{41}$ and $X^{43}$ are N, and $X^{42}$, $X^{44}$, and $X^{45}$ are CR, or $X^{42}$ and $X^{44}$ are N, and $X^{41}$, $X^{43}$, and $X^{45}$ are CR, or $X^{41}$ and $X^{45}$ are N, and $X^{42}$, $X^{43}$, and $X^{44}$ are CR;
each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and
$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

[12] The metal-organic framework according to [11], wherein the compound represented by Formula (1) is a compound represented by the following Formula (1-1):

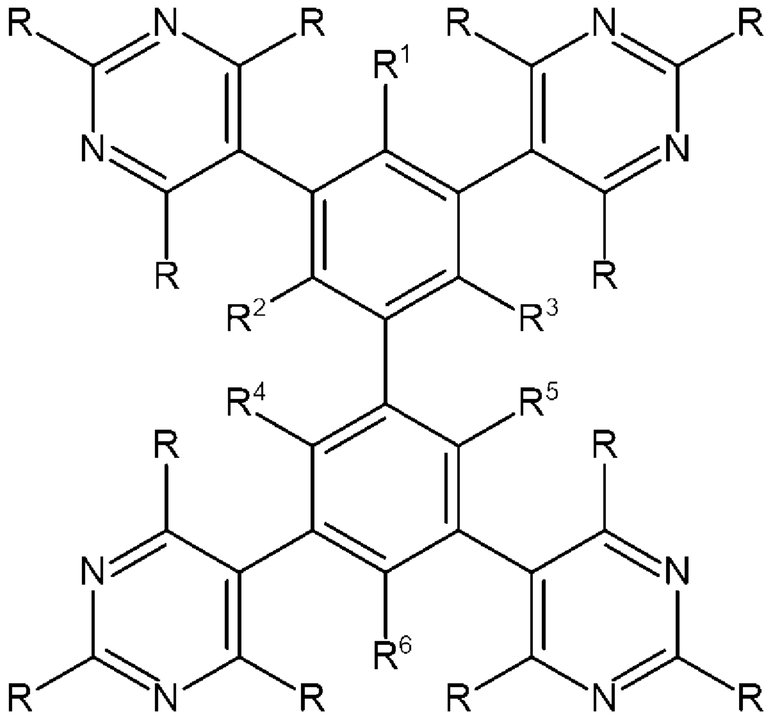

(1-1)

(in Formula (1-1),

each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and

$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

[13] The metal-organic framework according to [11] or [12], which comprises a halogen element as a constituent element.

[14] The metal-organic framework according to any one of [11] to [13], which has a composition formula represented by $Cu_4I_4L$, with the ligand being L.

[15] The metal-organic framework according to any one of [11] to [14], wherein

the constituent element contains copper, and

the ligand is coordinated to the copper by one nitrogen atom of two nitrogen atoms of a pyrimidine ring of the ligand.

[16] The metal-organic framework according to any one of [11] to [15], wherein

the constituent element contains copper and iodine, and

$Cu_4I_4$ is present in a cubane-type structure in the metal-organic framework.

[17] The metal-organic framework according to any one of [11] to [16], wherein

the constituent element contains copper and iodine,

$Cu_4I_4$ is present in a cubane-type structure in the metal-organic framework,

the metal-organic framework has a composition formula, which is represented by $Cu_4I_4L$, with the ligand being L, and

the ligand is coordinated to the copper by one nitrogen atom of two nitrogen atoms of a pyrimidine ring of the ligand.

[18] A carbon dioxide adsorbent comprising the metal-organic framework according to any one of [11] to [17].
[19] A compound represented by the following Formula (1):

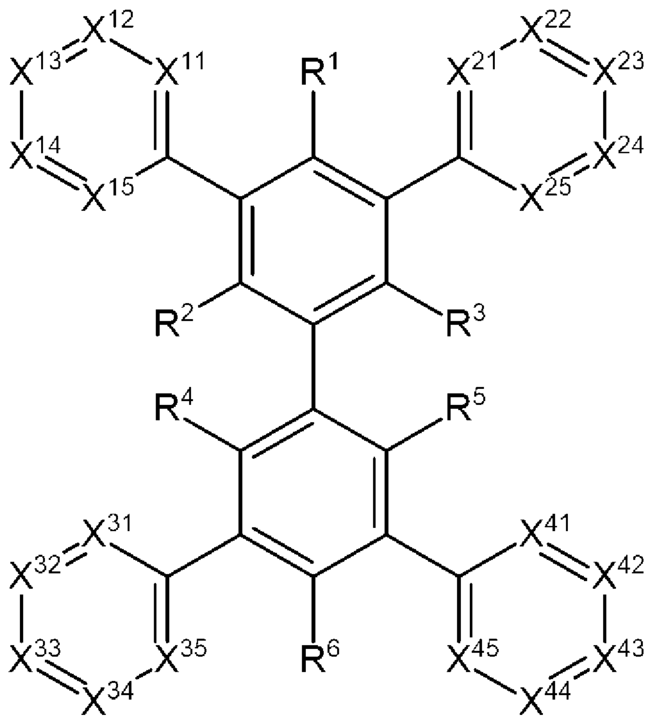

(1)

(in Formula (1),

$X^{11}$ and $X^{13}$ are N, and $X^{12}$, $X^{14}$, and $X^{15}$ are CR, or $X^{12}$ and $X^{14}$ are N, and $X^{11}$, $X^{13}$, and $X^{15}$ are CR, or $X^{11}$ and $X^{15}$ are N, and $X^{12}$, $X^{13}$, and $X^{14}$ are CR;
$X^{21}$ and $X^{23}$ are N, and $X^{22}$, $X^{24}$, and $X^{25}$ are CR, or $X^{22}$ and $X^{24}$ are N, and $X^{21}$, $X^{23}$, and $X^{25}$ are CR, or $X^{21}$ and $X^{25}$ are N, and $X^{22}$, $X^{23}$, and $X^{24}$ are CR;
$X^{31}$ and $X^{33}$ are N, and $X^{32}$, $X^{34}$, and $X^{35}$ are CR, or $X^{32}$ and $X^{34}$ are N, and $X^{31}$, $X^{33}$, and $X^{35}$ are CR, or $X^{31}$ and $X^{35}$ are N, and $X^{32}$, $X^{33}$, and $X^{34}$ are CR;
$X^{41}$ and $X^{43}$ are N, and $X^{42}$, $X^{44}$, and $X^{45}$ are CR, or $X^{42}$ and $X^{44}$ are N, and $X^{41}$, $X^{43}$, and $X^{45}$ are CR, or $X^{41}$ and $X^{45}$ are N, and $X^{42}$, $X^{43}$, and $X^{44}$ are CR;
each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and
$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

[20] The compound according to [19], which is a compound represented by the following Formula (1-1):

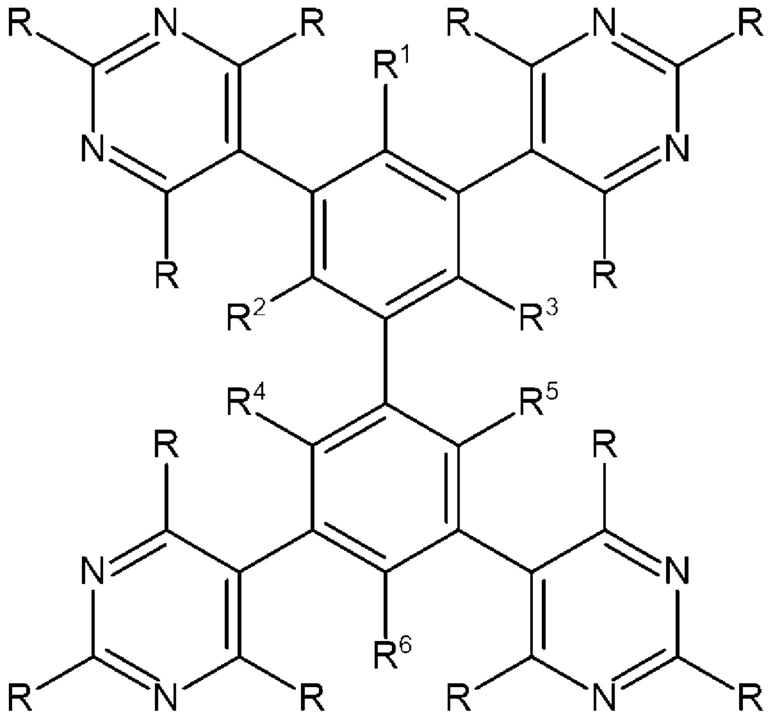

(1-1)

(in Formula (1-1),

each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and

$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

Advantageous Effects of Invention

According to the present invention, a novel carbon dioxide adsorbent that can capture carbon dioxide, a metal-organic framework that can be used for the carbon dioxide adsorbent, and a compound that can be used as a ligand for the metal-organic framework can be provided.

BRIEF DESCRIPTION OF THE DRAWINGS

FIG. 1 is a schematic diagram of an interpenetrating structure in which two frameworks interpenetrate each other.
FIG. 2 is a schematic diagram of a cubane-type structure.
FIG. 3 is a schematic diagram of the three-dimensional structure of an example of the metal-organic framework of the present invention (without carbon dioxide capture).
FIG. 4 is a schematic diagram of the three-dimensional structure of an example of the metal-organic framework of the present invention (with carbon dioxide capture).
FIG. 5A is a diagram showing the results of single crystal X-ray structure analysis of pyrimidine ligand (Lp) (Part 1).
FIG. 5B is a diagram showing the results of single crystal X-ray structure analysis of pyrimidine ligand (Lp) (Part 2).
FIG. 6 shows the FT-IR measurement results of the metal-organic framework [$Cu_4I_4Lp$] and the pyrimidine ligand (Lp) before solvent removal.
FIG. 7A is a diagram showing the single crystal structure of the metal-organic framework [$Cu_4I_4Lp$] before solvent removal (Part 1).
FIG. 7B is a diagram showing the single crystal structure of the metal-organic framework [$Cu_4I_4Lp$] before solvent

removal (Part 2).
FIG. 8A is another diagram showing the single crystal structure of the metal-organic framework [$Cu_4I_4Lp$] before solvent removal (Part 1).
FIG. 8B is another diagram showing the single crystal structure of the metal-organic framework [$Cu_4I_4Lp$] before solvent removal (Part 2).
FIG. 8C is another diagram showing the single crystal structure of the metal-organic framework [$Cu_4I_4Lp$] before solvent removal (Part 3).
FIG. 9A is yet another diagram showing the single crystal structure of the metal-organic framework [$Cu_4I_4Lp$] before solvent removal (Part 1).
FIG. 9B is yet another diagram showing the single crystal structure of the metal-organic framework [$Cu_4I_4Lp$] before solvent removal (Part 2).
FIG. 10 shows the PXRD measurement results of the metal-organic framework [$Cu_4I_4Lp$] before solvent removal.
FIG. 11 shows an adsorption isotherm of the metal-organic framework [$Cu_4I_4Lp$].
FIG. 12 is a diagram showing the single crystal structure of the metal-organic framework [$Cu_4I_4Lp$] after solvent removal.
FIG. 13 is a diagram showing the single crystal structure of the metal-organic framework [$Cu_4I_4Lp$] after $CO_2$ capture.
FIG. 14 shows the FT-IR measurement results of the metal-organic framework [$Cu_4I_4Lp$] before solvent removal [III], after solvent removal [II], and after $CO_2$ capture [I].

## DETAILED DESCRIPTION OF THE INVENTION

### (Carbon Dioxide Adsorbent)

The carbon dioxide adsorbent of the present invention comprises a metal-organic framework. The carbon dioxide adsorbent may consist of a metal-organic framework.
The metal-organic framework can capture and desorb carbon dioxide.
Isolated voids are formed inside the metal-organic framework by the metal-organic framework's three-dimensional structure.
The isolated voids are the space that can capture carbon dioxide and does not have a channel through which carbon dioxide can pass in an ordinary state.
In the metal-organic framework, although the three-dimensional structure of the framework changes during the process where carbon dioxide is captured within isolated voids and the process where carbon dioxide is released from isolated voids, the three-dimensional structure of the metal-organic framework when carbon dioxide is captured within the isolated voids is the same as when carbon dioxide is not captured within the isolated voids.
Here, the ordinary state refers to a state of being placed in the air at ordinary temperature (25°C) and ordinary pressure (1 atm).
Here, the channel refers to a passage between a void and another void or the outside through which carbon dioxide can pass.
Whether a specific molecule can pass through a passage between a void and another void or the outside can be determined based on the kinetic diameter of the molecule and the spatial size of the passage in terms of the possibility of passage of the molecule.
Here, the spatial size of the passage in terms of the possibility of passage of the molecule can be determined from the positions of the atoms forming the passage and the van der Waals radii of the atoms forming the passage. The passage is a space (referred to as a passage space) that is narrowed by a van der Waals radius from the position of each atom from the space surrounded by the atoms that constitute the passage.
In a case in which a sphere having a diameter corresponding to the kinetic diameter of a specific molecule can pass through the passage space, then that molecule will be able to pass through the passage.
In a case in which a sphere having a diameter corresponding to the minimum kinetic diameter of gaseous helium (2.57 Å) cannot pass through the passage space, then no molecules can pass through that passage.
The positions of atoms constituting the passages can be determined by single crystal X-ray structure analysis.
Metal-organic frameworks are also known as porous coordination polymers (PCPs).
A metal-organic framework has a framework. A framework is constructed by binding a ligand to a metal or metal compound that serves as a node via a coordinate bond and is composed of constituent elements and chemical bonds (mainly covalent bonds and coordinate bonds).
The present inventors have found a novel metal-organic framework that although the three-dimensional structure of the framework changes during the process where carbon dioxide is captured within isolated voids and the process where carbon dioxide is released from isolated voids, the three-dimensional structure of the metal-organic framework when carbon dioxide is captured within the isolated voids is the same as when carbon dioxide is not captured within the

isolated voids.

Meanwhile, in the novel metal-organic framework found by the present inventors, although the three-dimensional structure of the framework changes during the process where carbon dioxide is captured within isolated voids and the process where carbon dioxide is released from isolated voids, the three-dimensional structure of the metal-organic framework when carbon dioxide is captured within the isolated voids is the same as when carbon dioxide is not captured within the isolated voids

(hereinafter, this behavior in a metal-organic framework may be referred to as "no three-dimensional structure change before and after carbon dioxide capture"). Therefore, the above-described problems in gate-opening-type MOFs are less likely to occur. This point is advantageous when using metal-organic frameworks.

Here, the expression "the metal-organic framework's three-dimensional structure when carbon dioxide is captured within the isolated voids is the same as the metal-organic framework's three-dimensional structure when carbon dioxide is not captured within the isolated voids" means that for example, the rates of change between the lattice constants of the crystal structure of the metal-organic framework in which carbon dioxide is captured within an isolated voids ($a_1$ [Å], $b_1$ [Å], $c_1$ [Å], $\alpha_1$ [°], $\beta_1$ [°], $\gamma_1$ [°]) and the lattice constants of the crystal structure of the metal-organic framework in which carbon dioxide is not captured within the isolated voids ($a_2$ [Å], $b_2$ [Å], $c_2$ [Å], $\alpha_2$ [°], $\beta_2$ [°], $\gamma_2$ [°]) are within ±10%. These rates of change are preferably within ±5%, further preferably within ±3%, particularly preferably within ±1%. Here, the rate of change for each lattice constant is determined as follows.

- $$\text{Rate of change for a (\%)} = [(a_1 - a_2)/a_1] \times 100$$
- $$\text{Rate of change for b (\%)} = [(b_1 - b_2)/b_1] \times 100$$
- $$\text{Rate of change for c (\%)} = [(c_1 - c_2)/c_1] \times 100$$
- $$\text{Rate of change for } \alpha \text{ (\%)} = [(\alpha_1 - \alpha_2)/\alpha_1] \times 100$$
- $$\text{Rate of change for } \beta \text{ (\%)} = [(\beta_1 - \beta_2)/\beta_1] \times 100$$
- $$\text{Rate of change for } \gamma \text{ (\%)} = [(\gamma_1 - \gamma_2)/\gamma_1] \times 100$$

The lattice constants can be determined by single crystal X-ray structure analysis.

The present inventors believe that an important feature of the metal-organic framework with no three-dimensional structure change before and after carbon dioxide capture is the formation of an interpenetrating structure where two frameworks are mutually interwoven.

FIG. 1 is a schematic diagram of an interpenetrating structure (Christian S. Diercks, Omar M. Yaghi, Science 2017 Vol. 355, Issue 6328, eaal158). In the interpenetrating structure in FIG. 1, a first framework F1 and a second framework F2 interpenetrate each other. In other words, the second framework F2 is embedded within the first framework F1. The metal-organic framework forms such a structure (an interpenetrating structure in which two frameworks interpenetrate each other), and the relative positions of the two frameworks change, causing appropriate three-dimensional structure changes. Meanwhile, since two frameworks interpenetrate each other, changes in the relative positions of the two frameworks are limited. Therefore, three-dimensional structure changes are moderately restricted. This may cause three-dimensional structure changes that allow carbon dioxide to pass through an isolated void, while the metal-organic framework's three-dimensional structure when carbon dioxide is captured within an isolated voids can be the same as the metal-organic framework's three-dimensional structure when carbon dioxide is not captured within the isolated voids, according to the present inventors.

The present inventors also believe that it is essential that a metal-organic framework with no three-dimensional structure change before and after carbon dioxide capture is characterized by the elastic flexibility of a ligand.

The structure of the ligand is not rigid, and there are sites (bonds) in the skeleton of the ligand that cannot rotate but can twist (change the angle of one part of the molecule with respect to the rest of the molecule). This gives the ligand elastic flexibility. Ligand flexibility leads to three-dimensional structure changes in the metal-organic framework. It is thought that the elasticity of the flexibility allows the metal-organic framework to return to a stable structure when a factor that causes a change in the three-dimensional structure (such as pressure) is removed. This may cause three-dimensional structure changes that allow carbon dioxide to pass through isolated voids, while the metal-organic framework's three-dimensional structure when carbon dioxide is captured within isolated voids can be the same as the metal-organic framework's three-dimensional structure when carbon dioxide is not captured within the isolated voids, according to the present inventors.

Twisting (changing the angle of one part of the molecule with respect to the rest of the molecule) is likely to occur, for example, in a carbon-carbon bond between a nitrogen-containing aromatic heterocycle and an aromatic hydrocarbon ring in the ligand. The rotation of this carbon-carbon bond is highly restricted due to the physical proximity of the hydrogen or substituent bonded to the nitrogen-containing aromatic heterocycle and the hydrogen or substituent bonded to the aromatic hydrocarbon ring. Therefore, the twist originating from this carbon-carbon bond cannot freely rotate like the carbon-carbon single bond of a linear hydrocarbon group. In addition, once a factor that causes three-dimensional structure changes (e.g., pressure) is removed, the twist originating from this carbon-carbon bond returns to its original state to reduce the physical proximity of the hydrogen or substituent bonded to the nitrogen-containing aromatic heterocycle and the hydrogen or substituent bonded to the aromatic hydrocarbon ring. Thus, the twist is elastic.

In a case in which an aromatic hydrocarbon ring and a nitrogen-containing aromatic heterocycle are bonded via a carbon-carbon bond, the state where they are on the same plane is the highest energy state, i.e., the most unfavorable state. When a bulky substituent is present, the energy barrier can no longer be overcome, and rotation is inhibited. The angles formed by these planes vary depending on the presence or absence of substituents and the type of substituents. For example, in the case of a metal-organic framework [$Cu_4I_4Lp$] herein described in Examples, the angle between the plane of the pyrimidine ring and the plane of the benzene ring substituted with a methyl group is about 70°.

The metal-organic framework has a BET-specific surface area of, for example, 1 $m^2/g$ or less in a specific surface area measurement using $N_2$. In a metal-organic framework having pores capable of capturing carbon dioxide and $N_2$, a BET-specific surface area of 1 $m^2/g$ or less in specific surface area measurement using $N_2$ means that the pores are isolated voids.

The BET-specific surface area can be determined by measuring an adsorption isotherm using $N_2$. The adsorption isotherm can be measured using a gas adsorption measuring apparatus (e.g., fully automatic gas adsorption measuring apparatus BELSORP MAX from MicrotracBEL Corp.). Details of the measurement method are described in Examples.

For example, an element constituting an isolated void and carbon dioxide captured within the isolated void do not form a chemical bond in a metal-organic framework. The chemical bond herein is a covalent bond, an ionic bond, or a hydrogen bond.

Whether an element constituting an isolated void and carbon dioxide captured within the isolated void do not form a chemical bond can be determined based on the type of element constituting the isolated void and the size of the isolated void.

The size of the isolated voids can be obtained by calculating single crystal X-ray structure analysis results using the Mercury software from the Cambridge Crystallographic Data Centre (CCDC).

The metal-organic framework comprises, for example, at least one of groups II to XIV elements as a constituent element. The groups II to XIV elements are preferably, Zr, Cd, Ti, Cu, Zn, Fe, Cr, Ni, Co, Mo, Hf, Mg, Al, and Si, more preferably, Cu, Zr, Zn, and Cd.

The metal-organic framework may comprise, for example, a halogen element as a constituent element. Examples of a halogen element include fluorine, chlorine, bromine, and iodine.

The constituent element mentioned herein is not an element constituting a ligand.

The metal-organic framework comprises, for example, a compound having a nitrogen-containing aromatic heterocycle as a ligand. Examples of a nitrogen-containing aromatic heterocycle include a pyridine ring and a pyrimidine ring.

It is preferable that the composition formula of the metal-organic framework is represented by $Cu_4I_4L$, with a ligand being L.

It is preferable that the compound having a nitrogen-containing aromatic heterocycle is a compound represented by Formula (1) described later. Further, the compound represented by Formula (1) may be defined in the proviso described below. Regarding $X^{11}$ to $X^{15}$, $X^{21}$ to $X^{25}$, $X^{31}$ to $X^{35}$, and $X^{41}$ to $X^{45}$, an aspect is possible in which one of $X^{11}$ to $X^{15}$ is N and the others are CR, one of $X^{21}$ to $X^{25}$ is N and the others are CR, one of $X^{31}$ to $X^{35}$ is N and the others are CR, and one of $X^{41}$ to $X^{45}$ is N and the others are CR. Regarding $X^{11}$ to $X^{15}$, $X^{21}$ to $X^{25}$, $X^{31}$ to $X^{35}$, and $X^{41}$ to $X^{45}$, an aspect is possible in which $X^{13}$, $X^{23}$, $X^{33}$, and $X^{43}$ are N and the others are CR. R in CR has the same meaning as R in the proviso of the compound represented by Formula (1) described later.

The metal-organic framework described above is preferably the metal-organic framework of the present invention described in detail below.

(Metal-Organic Framework)

The metal-organic framework of the present invention comprises at least one of groups II to XIV elements as a constituent element and a compound represented by the following Formula (1) as a ligand.
The metal-organic framework may comprise a halogen element as a constituent element.
The constituent element mentioned herein is not an element constituting a ligand.
The groups II to XIV elements are preferably, Zr, Cd, Ti, Cu, Zn, Fe, Cr, Ni, Co, Mo, Hf, Mg, Al, and Si, more preferably, Cu, Zn, and Cd.
Examples of the halogen element include fluorine, chlorine, bromine, and iodine.
The composition formula of the metal-organic framework of the present invention is represented by, for example, $Cu_4I_4L$, with a ligand being L.
The ligand is, for example, coordinated to copper by one nitrogen atom of two nitrogen atoms of a pyrimidine ring of the ligand.
$Cu_4I_4$ is present in a cubane-type structure, for example, in the metal-organic framework of the present invention.
FIG. 2 is a schematic diagram of a cubane-type structure.
A cubane-type structure is a cube, with Cu or I at each vertex, and the line segments between Cu and I constitute edges. Cu and Cu are not adjacent, and neither are I and I. In other words, Cu and Cu exist only on a diagonal line, and I and I also exist only on a diagonal line.
Since Cu and I have different atomic sizes, the actual structure is not cubic but distorted. LCu-I-Cu is about 60°, and LI-Cu-I is about 110°.
Note that L in the figure represents ligand.
The crystal system of the metal-organic framework of the present invention in an ordinary state can be, for example, a tetragonal system.
The space group of the metal-organic framework of the present invention in an ordinary state can be, for example, $I4_1/a$.
A schematic diagram of the three-dimensional structure of an example of the metal-organic framework of the present invention will be described below.
FIG. 3 is a schematic diagram of the three-dimensional structure of an example of the metal-organic framework of the present invention (without carbon dioxide capture).
A first framework L1a, represented by light gray spheres (atoms) and rods (bonds), and a second framework L2a, represented by dark gray spheres (atoms) and rods (bonds), interpenetrate each other, thereby forming an interpenetrating structure in FIG. 3.
FIG. 4 is a schematic diagram of the three-dimensional structure of an example of the metal-organic framework of the present invention (with carbon dioxide capture).
In FIG. 4, a first framework L1a, represented by light gray spheres (atoms) and rods (bonds), and a second framework L2a, represented by dark gray spheres (atoms) and rods (bonds), interpenetrate each other, thereby forming an interpenetrating structure as in FIG. 3. Further, carbon dioxide, represented by three partially overlapping spheres (corresponding to C and O, respectively), is captured within the isolated voids formed by the two frameworks in FIG. 4.

<Compound Represented by Formula (1)>

The compound represented by Formula (1) is also the subject matter of the present invention

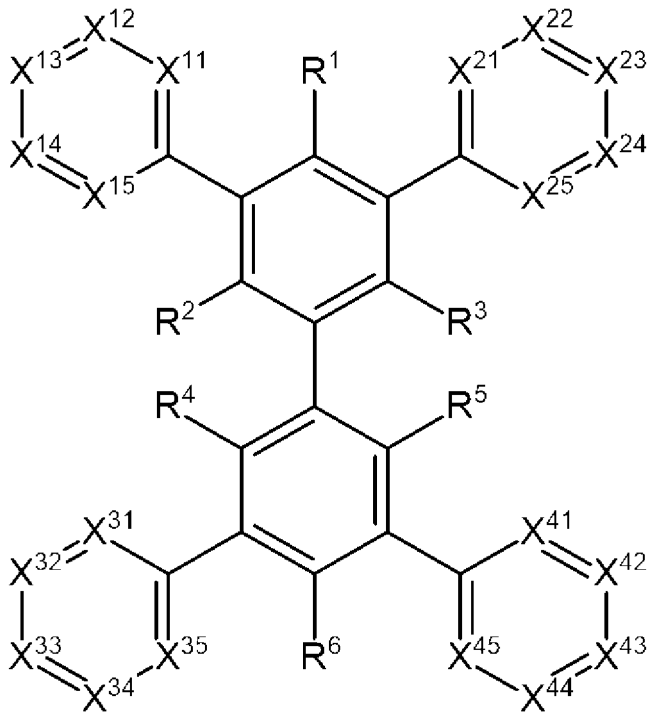

(1)

(In Formula (1),

$X^{11}$ and $X^{13}$ are N, and $X^{12}$, $X^{14}$, and $X^{15}$ are CR, or $X^{12}$ and $X^{14}$ are N, and $X^{11}$, $X^{13}$, and $X^{15}$ are CR, or $X^{11}$ and $X^{15}$ are N, and $X^{12}$, $X^{13}$, and $X^{14}$ are CR;
$X^{21}$ and $X^{23}$ are N, and $X^{22}$, $X^{24}$, and $X^{25}$ are CR, or $X^{22}$ and $X^{24}$ are N, and $X^{21}$, $X^{23}$, and $X^{25}$ are CR, or $X^{21}$ and $X^{25}$ are N, and $X^{22}$, $X^{23}$, and $X^{24}$ are CR;
$X^{31}$ and $X^{33}$ are N, and $X^{32}$, $X^{34}$, and $X^{35}$ are CR, or $X^{32}$ and $X^{34}$ are N, and $X^{31}$, $X^{33}$, and $X^{35}$ are CR, or $X^{31}$ and $X^{35}$ are N, and $X^{32}$, $X^{33}$, and $X^{34}$ are CR;
$X^{41}$ and $X^{43}$ are N, and $X^{42}$, $X^{44}$, and $X^{45}$ are CR, or $X^{42}$ and $X^{44}$ are N, and $X^{41}$, $X^{43}$, and $X^{45}$ are CR, or $X^{41}$ and $X^{45}$ are N, and $X^{42}$, $X^{43}$, and $X^{44}$ are CR;
each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and
$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom.)

Examples of a halogen atom include a fluorine atom, a chlorine atom, a bromine atom, and an iodine atom.

The alkyl group and the alkyl group in the alkyloxy group may be linear, branched, or cyclic. The number of carbon atoms in linear and branched alkyl groups is preferably from 1 to 30, more preferably from 1 to 12, particularly preferably from 1 to 4. In addition, the number of carbon atoms in a cyclic alkyl group is preferably from 3 to 30, more preferably from 3 to 12, particularly preferably from 3 to 10.

It is preferable that when $R^1$ to $R^6$ are an alkyl group or an alkyloxy group, the alkyl group and the alkyl group in the alkyloxy group are linear in that the alkyl group can impart appropriate steric hindrance to the benzene ring.

Examples of the alkyl group include methyl, ethyl, n-propyl, iso-propyl, n-butyl, iso-butyl, tert-butyl, n-pentyl, n-hexyl, cyclohexyl, n-heptyl, n-octyl, 2-ethylhexyl, n-nonyl, n-decyl, 3,7-dimethyloctyl, and n-lauryl groups. Among these, methyl, ethyl, n-propyl, iso-propyl, and n-butyl groups are preferable.

The number of halogen atom(s) in the alkyl group substituted with halogen atom(s) is not particularly limited.

Examples of the alkyl group substituted with halogen atom(s) include trifluoromethyl, pentafluoroethyl, perfluorobutyl, perfluorohexyl, and perfluorooctyl groups.

Examples of the alkyloxy group include methyloxy, ethyloxy, n-propyloxy, iso-propyloxy, n-butyloxy, iso-butyloxy, tert-butyloxy, n-pentyloxy, n-hexyloxy, cyclohexyloxy, n-heptyloxy, n-octyloxy, 2-ethylhexyloxy, n-nonyloxy, n-decyloxy, 3,7-dimethyloctyloxy, and lauryloxy groups. Among these, methyloxy, ethyloxy, n-propyloxy, iso-propyloxy, and n-butyloxy groups are preferable.

The number of halogen atom(s) in the alkyloxy group substituted with halogen atom(s) is not particularly limited.

Examples of the alkyloxy group substituted with halogen atom(s) include trifluoromethyloxy, pentafluoroethyloxy, perfluorobutyloxy, perfluorohexyloxy, perfluorooctyloxy, methyloxymethyloxy, and 2-methyloxyethyloxy groups.

The aryl group for R and $R^1$ to $R^6$ may be unsubstituted. The aryl group may be substituted with a halogen atom. The aryl group may be substituted with an alkyl group optionally substituted with a halogen atom. The aryl group may be substituted with an alkyloxy group optionally substituted with a halogen atom.

Examples of the (unsubstituted) aryl group include phenyl, 1-naphthyl, 2-naphthyl, 1-anthracenyl, 2-anthracenyl, and 9-anthracenyl groups.

Examples of the aryl group substituted with a halogen atom include a pentafluorophenyl group.

Examples of the aryl group substituted with an alkyl group optionally substituted with a halogen atom include a C1-C12 alkylphenyl group ("C1-C12" means that the number of carbon atoms is from 1 to 12, and the same applies hereafter).

Examples of the aryl group substituted with an alkyloxy group optionally substituted with a halogen atom include a C1-C12 alkyloxyphenyl group.

An aryl group is an atomic group obtained by removing one hydrogen atom from an aromatic hydrocarbon. Examples of such aromatic hydrocarbons include those having a condensed ring and those in which two or more selected from independent benzene rings and/or condensed rings are bonded directly or through a group such as vinylene.

R is preferably a hydrogen atom, a halogen atom, an alkyl group having 1 to 3 carbon atoms optionally substituted with a halogen atom, or an alkyloxy group having 1 to 3 carbon atoms optionally substituted with a halogen atom, more preferably a hydrogen atom.

$R^1$ to $R^6$ are each preferably a hydrogen atom, a halogen atom, an alkyl group having 1 to 3 carbon atoms optionally substituted with a halogen atom, or an alkyloxy group having 1 to 3 carbon atoms optionally substituted with a halogen atom, more preferably an alkyl group having 1 to 3 carbon atoms optionally substituted with a halogen atom.

Examples of $X^{11}$ to $X^{15}$, $X^{21}$ to $X^{25}$, $X^{31}$ to $X^{35}$, and $X^{41}$ to $X^{45}$ include the following combinations:

(i): a combination in which $X^{11}$, $X^{13}$, $X^{21}$, $X^{23}$, $X^{31}$, $X^{33}$, $X^{41}$, and $X^{43}$ are N, and $X^{12}$, $X^{14}$, $X^{15}$, $X^{22}$, $X^{24}$, $X^{25}$, $X^{32}$, $X^{34}$, $X^{35}$, $X^{42}$, $X^{44}$, and $X^{45}$ are C R;
(ii): a combination in which $X^{12}$, $X^{14}$, $X^{22}$, $X^{24}$, $X^{32}$, $X^{34}$, $X^{42}$, and $X^{44}$ are N, and $X^{11}$, $X^{13}$, $X^{15}$, $X^{21}$, $X^{23}$, $X^{25}$, $X^{31}$, $X^{33}$, $X^{35}$, $X^{41}$, $X^{43}$, and $X^{45}$ are CR; and
(iii): a combination in which $X^{11}$, $X^{15}$, $X^{21}$, $X^{25}$, $X^{31}$, $X^{35}$, $X^{41}$, and $X^{45}$ are N, and $X^{12}$, $X^{13}$, $X^{14}$, $X^{22}$, $X^{23}$, $X^{24}$, $X^{32}$, $X^{33}$, $X^{34}$, $X^{42}$, $X^{43}$, and $X^{44}$ are CR.

Among these, the combination (ii) is preferable because a regular three-dimensional structure is easily obtained. In other words, the compound represented by Formula (1) is preferably a compound represented by the following Formula (1-1).

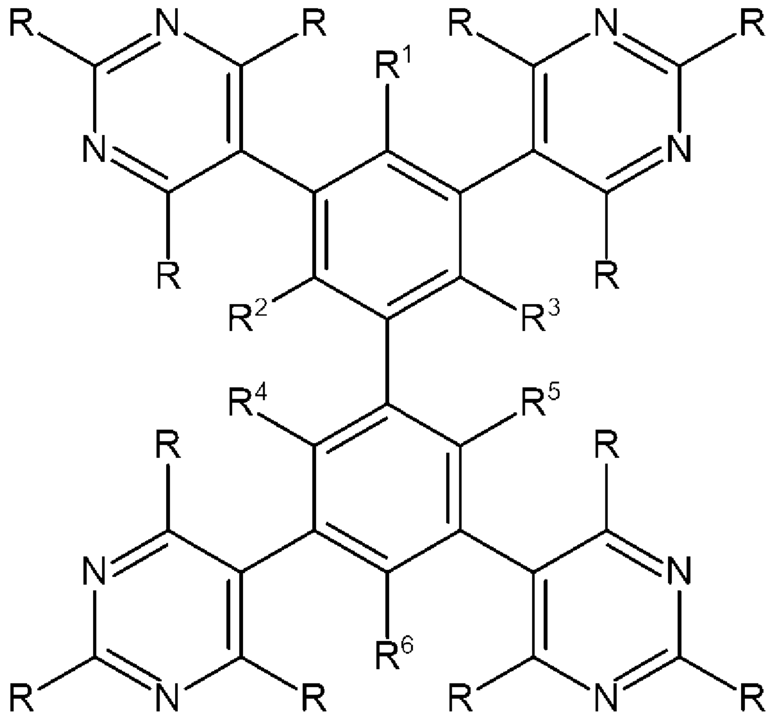

(1-1)

(In Formula (1-1),

each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and

$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom.)

<<Method for Producing Compound Represented by Formula (1) > >

The method for producing a compound represented by Formula (1) is not particularly limited. A production method according to the synthesis reaction in the scheme below can be mentioned.

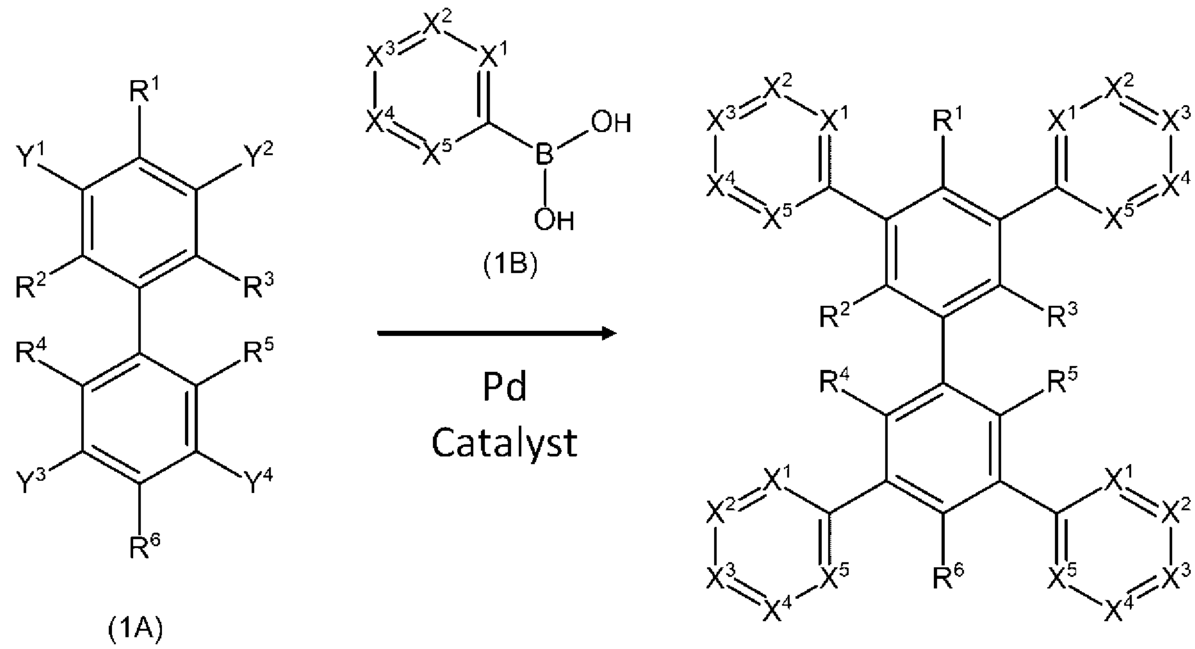


(1B)

(1A)

(1C)

(In Formula (1A), $Y^1$ to $Y^4$ are each a halogen atom.

In Formulas (1A) and (1C), $R^1$ to $R^6$ have the same meanings as $R^1$ to $R^6$ in Formula (1).

In Formula (1B) and Formula (1C), $X^1$ to $X^5$ have the same meanings as $X^{11}$ to $X^{15}$, $X^{21}$ to $X^{25}$, $X^{31}$ to $X^{35}$, and $X^{41}$ to $X^{45}$ in Formula (1).)

The synthesis reaction in the above scheme is a so-called Suzuki-Miyaura coupling reaction.

Examples of a palladium catalyst used in the reaction include [1,1'-bis(diphenylphosphino)ferrocene]palladium(II)dichloride ($PdCl_2$(dppf)), tetra kiss(triphenylphosphine)palladium ($Pd(PPh_3)_4$), bis(triphenylphosphine)dichloropalladium ($Pd(PPh_3)_2Cl_2$), bis(benzylidene acetone) palladium ($Pd(dba)_2$), tris(benzylidene acetone)dipalladium ($Pd_2(dba)_3$), bis(tritert-butylphosphine)palladium ($Pd(P\text{-}t\text{-}Bu_3)_2$), palladium acetate ($Pd(OAc)_2$), and chloro[(tri-tert-butylphosphine)-2-(2-aminobiphenyl)]palladium (II)(($tBu_3P$)Pd G2). These catalysts may be used with known suitable ligands. The amount of catalyst used may be a so-called catalytic amount. It is preferably 20% by mole or less, particularly preferably 10% by mole or less, with respect to the amount of a compound represented by Formula (1B). In a case in which a ligand is used, the amount of ligand used may be a so-called catalytic amount. It is preferably 20% by mole or less, particularly preferably 10% by mole or less, with respect to the amount of a compound represented by Formula (1B).

In addition, a base is also used in the synthesis reaction. Examples of the base include hydroxides, alkoxides, fluoride salts, carbonates, phosphates, and fluoride salts.

Examples of hydroxides include sodium hydroxide, potassium hydroxide, and cesium hydroxide.

Examples of alkoxides include sodium tert-butoxy and potassium tert-butoxy.

Examples of fluoride salts include lithium fluoride, potassium fluoride, and cesium fluoride.

Examples of carbonates include lithium carbonate, sodium carbonate, potassium carbonate, cesium carbonate, sodium hydrogen carbonate, and potassium hydrogen carbonate.

Examples of phosphates include potassium phosphate.

Examples of amines include trimethylamine, triethylamine, diisopropylamine, n-butylamine, and diisopropylethylamine.

Among these, from the perspective of efficiently obtaining the target product, the base is preferably a carbonate or phosphate, more preferably potassium carbonate or cesium carbonate.

The amount of base used is preferably from 1 to 20 mol, more preferably from 2 to 10 mol, with respect to 1 mol of the compound of Formula (1B).

The solvent used in the synthesis reaction is not particularly limited as long as it does not adversely affect the reaction. Specific examples thereof include aliphatic hydrocarbons, halogenated aliphatic hydrocarbons, aromatic hydrocarbons, ethers, amides, lactams, lactones, alcohols, urea derivatives, sulfoxides, and water.

Examples of aliphatic hydrocarbon include pentane, n-hexane, n-octane, n-decane, and decalin.

Examples of halogenated aliphatic hydrocarbons include Chloroform, dichloromethane, dichloroethane, and carbon tetrachloride.

Examples of aromatic hydrocarbons include benzene, nitrobenzene, toluene, o-xylene, m-xylene, p-xylene, and mesitylene.

Examples of ethers include diethyl ether, diisopropyl ether, tert-butyl methyl ether, tetrahydrofuran (THF), dioxane, 1,2-dimethoxyethane, and 1,2-diethoxyethane.

Examples of amides include N,N-dimethylformamide (DMF) and N,N-dimethylacetamide.

Examples of lactams include N-methylpyrrolidone.

Examples of lactones include $\gamma$-butyrolactone.

Examples of alcohols include methanol, ethanol, and propanol.

Examples of urea derivatives include N,N-dimethylimidazolidinone and tetramethylurea.

Examples of sulfoxides include dimethyl sulfoxide, sulfolane, and nitriles (acetonitrile, propionitrile, butyronitrile).

These may be used singly or in a combination of two or more.

The charging amounts of the compound represented by Formula (1A) and the compound represented by Formula (1B) are such that the compound represented by Formula (1B) is preferably from 4 to 10 mol, more preferably from 4.2 to 10 mol, with respect to 1 mol of the compound represented by Formula (1A) for efficient proceeding of the cyclization reaction.

The reaction temperature of the synthesis reaction is appropriately set within the range from the melting point to the boiling point of the solvent, considering the type and amount of the raw material compounds and catalysts used. It is generally from about 0°C to 200°C, preferably 20°C to 100°C.

The reaction time for the synthesis reaction cannot necessarily be defined because it varies depending on the raw material compounds used, the reaction temperature, and the like; however, it is usually from about 1 to 72 hours.

The synthesis reaction is preferably carried out in a state where nitrogen is circulated in a reaction container.

<Method for Producing Metal-Organic Framework>

The method for producing metal-organic framework is not particularly limited. A known method can be adopted as a manufacturing method of MOF. Examples thereof include the one-pot synthesis method (e.g., self-assembly method, solvothermal method, microwave irradiation method, ionothermal method, or high-throughput method), the stepwise synthesis method (e.g., metal-organic node framework precursor complex method, complex ligand method, in-situ sequential synthesis method, or post-synthesis modification method), the sonochemical synthesis method, and the mechanochemical synthesis method.

Among these, the solvothermal method is preferable in that stable thermodynamic products are obtained.

A metal-organic framework can be produced using the solvothermal method with reference to literature (e.g., Shi-Bin Ren, et al. CrystEngComm, 2009, 11, 1834-1836).

An example of the method for producing a metal-organic framework comprising a compound represented by Formula (1), copper, and iodine using the solvothermal method will be described below.

In the solvothermal method, for example, a mixture of a compound represented by Formula (1), copper(I) iodide, potassium iodide, and a solvent is heated. Potassium iodide is used to improve the solubility of CuI in the solvent. Therefore, potassium iodide may not be used depending on the type of solvent and the type of ligand.

The mixing ratio of a compound (L) represented by Formula (1) and copper(I) iodide (CuI) in producing a metal-organic framework is not particularly limited. However, the molar ratio (L: CuI) is preferably from 1:4 to 1:10, more preferably from 1:4 to 1:6.

The amount of potassium iodide used when producing a metal-organic framework is not particularly limited. However, it is preferably from 10 to 100 mol, more preferably from 30 to 80 mol with respect to 1 mol of copper(I) iodide.

When producing a metal-organic framework, a modulator may be used to promote crystallization, if necessary. Examples of a modulator include triphenylphosphine, pyridinium hydrochloride, and isoquinoline.

The amount of modulator used when producing a metal-organic framework is not particularly limited. However, it is preferably from 0.5 to 10 equivalents, more preferably from 1 to 5 equivalents with respect to the amount of the compound represented by Formula (1).

Examples of the solvent include, but are not limited to, N,N-dimethylformamide (DMF), N,N-diethylformamide (DEF), acetonitrile, formic acid, acetic acid, methanol, ethanol, water, and a mixed solvent of two or more thereof.

Among these, a mixed solvent of acetonitrile, ethanol, and water is preferable.

The amount of solvent used when producing a metal-organic framework is not particularly limited.

During heating, a raw material solution may be placed in any sealed container, or the raw material solution may be refluxed.

The heating temperature is not particularly limited. For example, it may not less than 100°C or not less than 120°C from the perspective of increasing reactivity, and it may be not more than 150°C from the perspective of preventing steam leakage during reaction.

The heating time is not particularly limited and can be adjusted as appropriate depending on the heating

temperature. The heating time may be, for example, 6 hours or more, 10 hours or more, 12 hours or more, 18 hours or more, 24 hours or more, 30 hours or more, 36 hours or more, 42 hours or more, 48 hours or more, 54 hours or more, or 60 hours or more, and it may be 96 hours or less, 84 hours or less, 72 hours or less, 60 hours or less, 48 hours or less, 24 hours or less, 12 hours or less, or 10 hours or less from the perspective of completing the reaction completely.

Further, after the reaction is completed, the obtained product may be appropriately post-treated.

For example, filtration of the product obtained may be carried out as post-treatment. Further, if necessary, a poor solvent or the like may be added to the filter cake obtained by filtration, and the mixture may be dispersed at room temperature or by heating as appropriate and then filtered again. The poor solvent that can be used herein may be a solvent in which the desired metal-organic framework is unlikely to be dissolved. For example, water, acetonitrile, hexane, ethanol, dimethylformamide, or the like can be used. In addition, the temperature when heating may be, for example, 40°C or more, 50°C or more, 60°C or more, 70°C or more, or 80°C or more, and it may be 100°C or less, 90°C or less, or 80°C or less. The heating time when heating may be 1 hour or more, 2 hours or more, 6 hours or more, 10 hours or more, or 12 hours or more, and it may be 24 hours or less or 16 hours or less.

Further, the desired metal-organic framework can be obtained by appropriately drying the filter cake obtained by filtration or re-filtration. Here, the drying may be performed under normal pressure or reduced pressure, but from the perspective of improving efficiency, drying is preferably performed under reduced pressure. The temperature when drying may be, for example, 20°C or more, 25°C or more, 40°C or more, 50°C or more, or 60°C or more, and it may be 100°C or less, 90°C or less, 80°C or less, or 60°C or less. The drying time when drying may be, for example, 1 hour or more, 2 hours or more, 6 hours or more, 10 hours or more, or 12 hours or more, and it may be 24 hours or less or 16 hours or less.

<Intended Use>

The intended use of the metal-organic framework is not particularly limited. However, as it has an excellent ability to capture carbon dioxide, it is preferably used as a carbon dioxide adsorbent.

The carbon dioxide adsorbent can also be suitably used in a carbon dioxide storage system that can store carbon dioxide.

EXAMPLES

The present invention will be specifically described below with reference to Examples, but the present invention is not limited to these Examples.

<NMR Measurement Conditions>

The $^1$H-NMR spectrum (400 MHz) and $^{13}$C-NMR spectrum (400 MHz) were measured by JNM-ECA400II from JEOL. Measurement samples were dissolved in deuterated chloroform ($CDCl_3$), to which tetramethylsilane (TMS) was added. Chemical shifts were derived using TMS as a standard (δ0.0 ppm).

<Single Crystal X-ray Structure Analysis Conditions>

Single-crystal X-ray diffraction data of a crystal of the pyrimidine ligand (Lp) and a crystal of the metal-organic framework [$Cu_4I_4$Lp] containing a solvent (MeCN: acetonitrile) in the pores were analyzed by the X-ray structure analyzer VariMax with Saturn from Rigaku. Using a low-temperature cooling device, Rigaku GNNP, the single crystal was cooled by nitrogen blowing at 123 K. The X-rays used for irradiation were MoKo rays (λ = 0.71075 Å) extracted by a graphite monochromator, and the detector was a two-dimensional CCD detector. The measured diffraction data were analyzed using the CrysAlisPro software from Rigaku.

Single crystal X-ray diffraction data on the crystal of the metal-organic framework [$Cu_4I_4$Lp] from which the solvent in the pores was removed (i) and the crystal of the metal-organic framework [$Cu_4I_4$Lp] that captured $CO_2$ (ii) were measured with BL-5A of the Photon Factory (PF) of the Institute of Materials Structure Science (IMSS) of the High Energy Accelerator Research Organization (KEK). The single crystals were cooled to 90 K with a Rigaku CryoCooler, irradiated with synchrotron radiation (λ = 0.7500 Å), and the diffraction pattern was detected with Dectris Pilatus 3 S6M. The measured diffraction data were integrated by the software XDS. Immediately after the crystals that captured $CO_2$ were taken out into the air from the $CO_2$ atmosphere, the crystals were immersed in oil for measurement.

<Gas Adsorption Measurement Conditions>

To measure the adsorption/desorption isotherms for the capture and desorption of nitrogen (77K and 298K)

and carbon dioxide (273K and 298K), a fully automatic gas adsorption measuring apparatus BELSORP MAX from MicrotracBEL Corp. was used. Samples were ground in a mortar, and about 60 mg was placed in a glass measuring container attached to a measuring apparatus. The inside of the container was vacuumed using a rotary pump and a turbo molecular pump, and the solvent inside the pores was removed by heating at 473 K for 12 hours at 1 kPa or less.

Without removing the container from the apparatus, the adsorption amount (amount of gas captured) was measured by a constant volume gas adsorption method. This is a method in which a fixed volume of gas is introduced into a measurement container, and the adsorption amount (amount of gas captured) is calculated by detecting the change in gas pressure. An adsorption isotherm was created by increasing the amount of gas stepwise, and a desorption isotherm related to gas desorption was obtained by reducing the pressure by vacuuming. After heating at 473 K for 2 hours at 1 kPa or less, the measurement container was removed from the apparatus, and the mass of the sample (adsorbate) after solvent removal was determined by weighing using a precision balance. Furthermore, at 77K, the measured temperature was maintained by filling a Dewar bottle with liquid nitrogen, and at 273K or 298K, by circulating an antifreeze solution filled in a water tank using an open cooling circulator. The analysis program BEL MASTER (trademark) was used to analyze the experimental results.

<Infrared Absorption Spectrum>

Infrared absorption spectra were measured with a Fourier transform infrared spectrophotometer Nicolet (trademark) iS (trademark) 50 from ThermoFisher Scientific. Measurement was performed by a diffuse reflection method using a liquid nitrogen-cooled MCT-A detector. Samples were diluted with potassium bromide (KBr), and KBr was used as a background.

<Powder X-ray Diffraction (PXRD) Measurement>

For the powder X-ray diffraction measurement, the fully automatic multi-purpose X-ray diffractometer SmartLab from Rigaku was used. At room temperature, each powder sample was irradiated with CuK characteristic X-rays ($\lambda$ = 1.5418 Å), and the diffraction pattern was detected while rotating the sample using a D/teX Ultra (1D) detector.

<Elemental Analysis>

A macro organic element analyzer vario MICRO cube from Elementar was used for elemental analysis.

(Example 1)

Synthesis of the pyrimidine ligand (Lp) was carried out based on the following synthesis scheme.

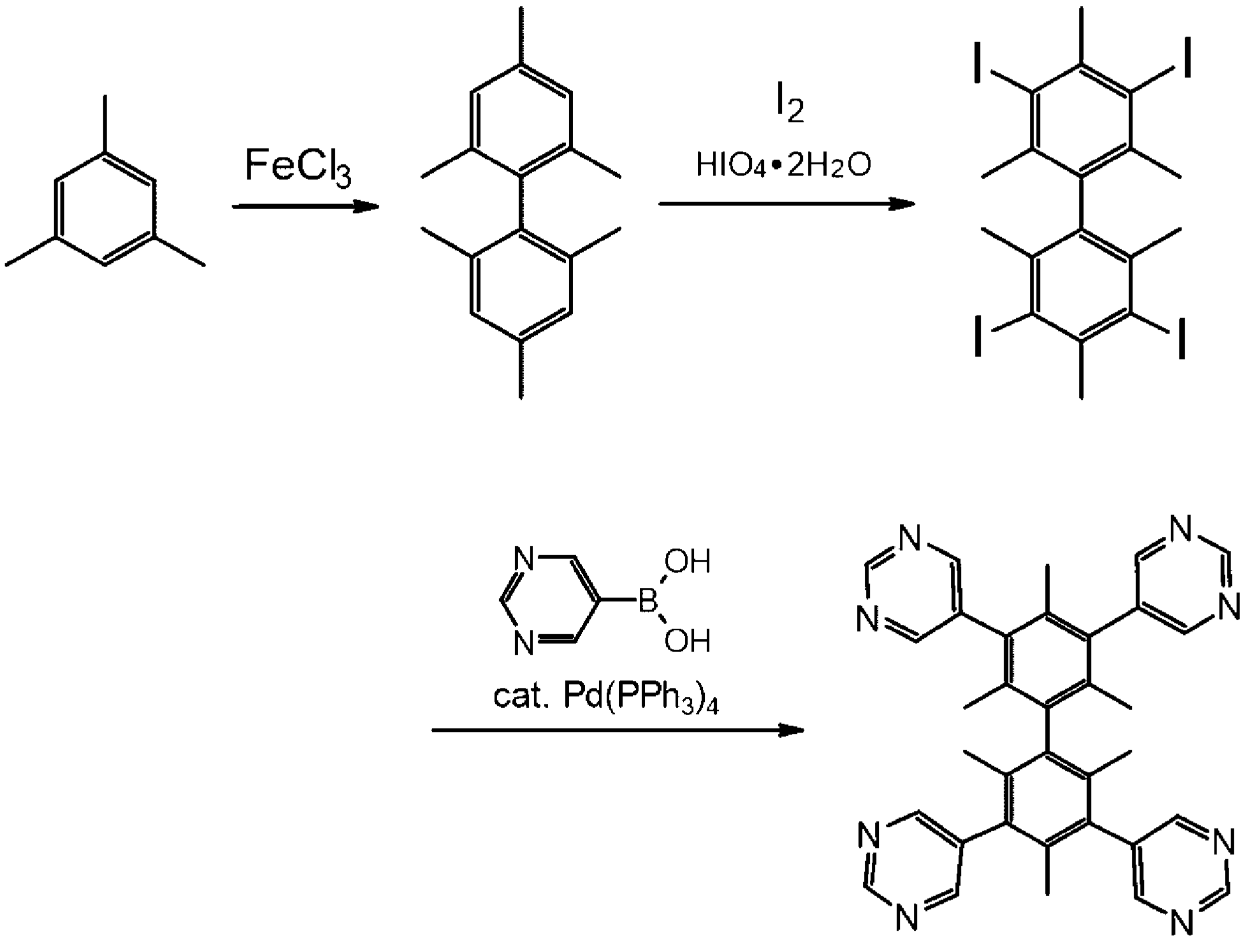


<Synthesis of 2,2',4,4',6,6'-hexamethyl-1,1'-biphenyl>

Iron(III) chloride hexahydrate (59 g, 0.090 mol) was added to mesitylene (100 mL, 0.18 mol) in a three-necked flask, and the mixture was stirred for 4 hours under a nitrogen atmosphere. The resulting reaction solution was quenched by pouring into ice water and transferred to a separatory funnel. The organic layer was taken out, washed twice with water, and then dried over sodium sulfate. This was transferred to a 100 mL flask, and mesitylene was distilled off by vacuum distillation. When the residue after distillation was cooled with ice water, crystals were precipitated. Crystals were taken out by suction filtration and washed with acetone, thereby obtaining a pale yellow powder. The yield was 12%.

$^1$H NMR (JEOL 400 MHz $CDCl_3$, TMS standard): δ6.93 (s,4H,Ha), 2.31 (s,6H,Hb), 1.85 (s,12H,Hc)

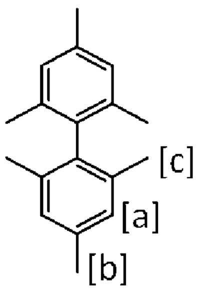

<Synthesis of 3,3',5,5'-tetraiodo-2,2',4,4',6,6'-hexamethyl-1,1'-biphenyl>

A mixed solvent of acetic acid (120 mL), water (24 mL), and sulfuric acid (3.6 mL) was added to a 300 mL flask containing 2,2',4,4',6,6'-hexamethyl-1,1'-biphenyl (2.0 g, 8.5 mmol), iodine (3.5 g, 13.6 mmol), and periodic acid (1.6g,6.8 mmol). The mixture was heated under reflux at 90°C for three days. The resulting mixture was poured into water, and the solid precipitate was removed by suction filtration and washed with water. The pink solid was dissolved in chloroform

(100 mL), the solution was washed with a saturated aqueous sodium thiosulfate solution, and the iodine was removed by separation. The organic layer was dried with magnesium sulfate, and an orange solid was obtained by drying under reduced pressure. The solid was washed with ethyl acetate and filtered under suction, thereby obtaining the desired product as a white powder. The yield was 71%.

$^1$H NMR (JEOL 400 MHz $CDCl_3$, TMS standard): δ3.03 (s,6H,Ha), 2.03 (s,12H,Hb)

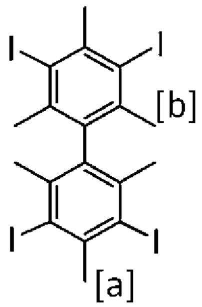

<Synthesis of (5,5',5'',5'''-(2,2',4,4',6,6'-hexamethyl-[1,1'-biphenyl]-3,3',5,5'-tetrayl)tetra-pyrimidine)(pyrimidine ligand (Lp)>

3,3',5,5'-tetraiodo-2,2',4,4',6,6'-hexamethyl-1,1'-biphenyl (0.52 g, 0.70 mmol), 5-pyrimidylboronic acid (0.41 g, 3.3 mmol), tetrakistriphenylphosphine palladium (0.24 g, 0.22 mmol), and potassium carbonate (2.84 g, 21 mmol) were added to a 50 mL Schlenk tube. The mixture was treated alternately by vacuuming and nitrogen ventilation, which was repeated three times. The atmosphere in the reaction was replaced with nitrogen.

Toluene (30 mL), ethanol (20 mL), and distilled water (10 mL), which had been degassed by bubbling nitrogen gas, were added, and the mixture was heated under reflux at 80°C for two days under nitrogen flow. The solvent was removed by vacuum drying, and the solid was dissolved in chloroform and distilled water. The organic layer was taken out by a liquid separation operation and washed with saturated brine. The organic layer was dried over magnesium sulfate and dried under reduced pressure, thereby obtaining a crude product. The crude product was dissolved in a small amount of chloroform and purified by silica gel column chromatography using ethyl acetate/triethylamine = 100/1 (volume ratio) as a developing solvent. The target product obtained was a white powder, and the yield was 69%.

$^1$H NMR (JEOL 400 MHz $CDCl_3$, TMS standard): δ9.25 (s,4H,Ha), 8.64 (s,8H,Hb), 1.75 (s,6H,Hc), 1.72 (s,12H,Hd),

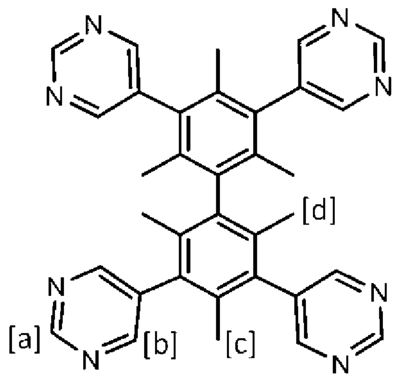

$^{13}$C NMR (JEOL 400 MHz $CDCl_3$, TMS standard): δ157.8 (A), 157.3 (B), 133.6-138.7 (C-G), 20.0 (H), 18.9 (I)

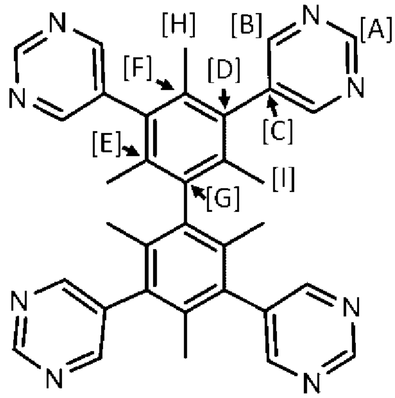

Elemental analysis. Calcd for [$C_{34}H_{30}N_8 \cdot 0.2SCH_3COOC_2H_5$]: C, 73.40; H, 5.63; N, 19.56.
Found: C, 73.40; H, 5.54; N, 19.34.

<Single Crystal X-ray Structure Analysis>

Pyrimidine ligand (Lp) was dissolved in chloroform in a vial, covered with KimWipe, and chloroform was evaporated for about half a day, thereby obtaining a single crystal of pyrimidine ligand (Lp).
Single crystals were measured using a micro single crystal X-ray structure analyzer (Rigaku VariMax with Saturn), and the single crystal structure was successfully analyzed from diffraction. Table 1 shows the results.

Table 1

| | |
|---|---|
| Empirical formula | $C_{37}H_{35}Cl_{8.06}N_8$ |
| Formula weight | 877.45 |
| Temperature/K | 123 |
| Crystal system | *orthorhombic* |
| Space group | *Pna2₁* |
| a/Å | 18.118(1) |
| b/Å | 19.201(1) |
| c/Å | 11.9888(8) |
| $\alpha$/° | 90 |
| $\beta$/° | 90 |
| $\gamma$/° | 90 |
| Volume/$Å^3$ | 4170.7(5) |
| Z | 4 |
| F (000) | 1800.0 |
| Radiation | MoKo ($\lambda$ =0.71073) |
| $2\theta$ ranqe for data collection/° | 3.09 to 62.392 |
| Reflections collected | 58408 |
| Independent reflections | 12584 [$R_{int}$ = 0.2184, $R_{sigma}$ = 0.2905] |
| Goodness-of-fit on $F^2$ | 0.98 |
| Final R indexes [I>2$\sigma$ (I)] | $R_1$=0.1125, $wR_2$=02309 |
| Final R indexes [all data] | $R_1$=0.3050, $wR_2$=0.3231 |

The results of single crystal X-ray structure analysis are shown in the figures. FIGS. 5A and 5B show the results. FIG. 5A shows a unit cell and FIG. 5B shows the structure of one molecule.

It was confirmed that the desired compound was obtained also by the single crystal structure. Furthermore, the dihedral angle of the two central aromatic rings was measured to be 92.15°, which is approximately orthogonal. This revealed that a ligand similar to d symmetry was obtained.

(Example 2)

<Synthesis of Metal-Organic Framework [$Cu_4I_4$Lp]>

Acetonitrile (5.4 mL), distilled water (3.6 mL), and ethanol (1 mL) were sequentially added to a PTFE sample decomposition container containing a pyrimidine ligand (Lp) (11 mg, 0.020 mmol), copper(I) iodide (19 mg, 0.10 mmol), potassium iodide (0.83 g, 5.0 mmol), and triphenylphosphine (5.2 mg, 0.020 mmol). The mixture was heated in an oven at 140°C for 64 hours. After heating, the temperature was slowly lowered in the oven, and after more than half a day, the container was taken out of the oven. The precipitate was taken out by suction filtration and washed with dimethylformamide, water, and acetonitrile. Thus, yellow prism crystals were obtained. In this state, the solid was a mixture. A pure metal-organic framework [$Cu_4I_4$Lp] was obtained by immersing the solid in a liquid mixture of 4 mL of dichloromethane and 3 mL of dibromomethane and taking out only the settled crystals. The yield was 58% based on the ligand.

Elemental analysis. Calcd for {$Cu_4I_4$[$C_{34}H_{30}N_8$(Lp)]·2.21 ($CH_3CN$)·1.17 ($C_2H_5OH$)}: C, 33.53; H, 3.01; N, 9.75. Found: C, 33.53; H, 2.76; N, 9.75.

<Characterization Before Solvent (acetonitrile) Removal>

Acetonitrile existed in the pores of the metal-organic framework [$Cu_4I_4$Lp] immediately after synthesis. FT-IR measurement, single crystal X-ray structure analysis, PXRD measurement, and adsorption isotherm measurement were performed in this state.

<<FT-IR Measurement>>

The solid crystal of the metal-organic framework [$Cu_4I_4$Lp] was measured using an FT-IR spectrophotometer (Nicolet (trademark) iS (trademark) 50 FT-IR). FIG. 6 shows the results together with the results for the pyrimidine ligand (Lp). Note that the results were obtained before removing the solvent (acetonitrile) in the crystals.

When comparing the IR spectra of the pyrimidine ligand (Lp) and the metal-organic framework [$Cu_4I_4$Lp], a new peak ([A]) was observed in the network around 2200 $cm^{-1}$. This is thought to be due to the stretching vibration of C=N bonds and is presumed to originate from acetonitrile entering the isolated voids (pore).

<<Single Crystal X-ray Structure Analysis>>

Single crystals obtained by the method described above were measured using a micro single crystal X-ray structure analyzer (Rigaku VariMax with Saturn), and the crystal structure was successfully analyzed from diffraction. Table 2 shows the analysis results in detail. Note that the results were obtained before removing the solvent (acetonitrile) in the crystals.

[Table 2]

| | [$Cu_4$I4Lp] (before acetonitrile removal) |
|---|---|
| Empirical formula | $C_{38}H_{30}Cu_4I_4N_{10}$ |
| Formula weight | 1388.48 |
| Temperature/K | 123 |
| Crystal system | *tetragonal* |
| Space group | *$14_1/a$* |
| a/Å | 12.3671(1) |
| b/Å | 12.3671(1) |
| c/Å | 28 9126(7) |

(continued)

| | $[Cu_4I4Lp]$ (before acetonitrile removal) |
|---|---|
| α/° | 90 |
| β/° | 90 |
| γ/° | 90 |
| Volume/Å $^3$ | 4422.0(1) |
| Z | 4 |
| $\rho_{calc}$g/cm$^3$ | 2.086 |
| μ/mm$^{-1}$ | 4.730 |
| F (000) | 2624.0 |
| Crystal size/mm$^3$ | 0.257 x 0.114 x 0.094 |
| Radiation | MoKo (λ =0.71073) |
| 2θ ranqe for data collection/° | 6.59 to 55.096 |
| Reflections collected | 27461 |
| Independent reflections | 2540 [$R_{int}$ = 0.0673, $R_{sigma}$ = 0.0216] |
| Data/restraints/parameters | 2540/24/142 |
| Goodness-of-fit on F$^2$ | 1.23 |
| Final R indexes [I>=2a (I)] | $R^1$ =0.0751, w$R_2$ = 0.1819 |
| Final R indexes [all data] | $R^1$ =0.0764, w$R_2$ = 0.1825 |

The results of single crystal X-ray structural analysis of the obtained metal-organic framework $[Cu_4I_4Lp]$ are shown in FIGS. 7A to 7B, FIGS. 8A to 8C, and FIGS. 9A to 9B.

The metal-organic framework $[Cu_4I_4Lp]$ had a cubane-type connector as a metal connector. The cubane-type connector had a four-coordinate diamond structure like the ligand, and the structure as a whole formed a three-dimensional diamond structure. In addition, disordered acetonitrile was present as a solvent in the pores. The packing structure is the same as shown in FIGS. 8A to 8C, but the solvent is not shown. It was found that pores were present where the solvent was. Further, this structure has an interpenetrating structure in which two frameworks interpenetrate each other.

When the size of the pore was calculated using Mercury from CCDC, the size of the pores was a space of 5.4 Å × 4.9 Å × 4.9 Å, and the porosity was 12%. Furthermore, the passage between the space and other spaces was large enough that a sphere having a diameter equivalent to a kinetic diameter (2.57 Å) of gaseous helium could not pass through. Therefore, apparently, in the obtained metal-organic framework $[Cu_4I_4Lp]$, there are no passages through which substances can pass between the pores and other pores or between the pores and the outside. In other words, the pores of the obtained metal-organic framework $[Cu_4I_4Lp]$ were isolated voids. This is believed to be due to the bulky structure of the ligand and the interpenetrating structure of the two frameworks.

<<PXRD>>

Powder X-ray diffraction (PXRD) measurement of the metal-organic framework $[Cu_4I_4Lp]$ was performed. FIG. 10 shows the results. Note that the results were obtained before removing the solvent (acetonitrile) in the crystals.

The PXRD pattern is the same as shown in FIG. 10, and almost matches the simulation from a single crystal structure using Mercury from CCDC. Since no other peaks were observed, it was found that a uniform structure was obtained.

<<Gas Adsorption Isotherm>>

Adsorption isotherms for gas trapping of the metal-organic framework $[Cu_4I_4Lp]$ were determined. FIG. 11 shows the results. Note that the results were obtained after removing the solvent (acetonitrile) in the crystals. Note that

the solvent is removed by an operation during gas adsorption measurement (operation of vacuuming the inside of the container using a rotary pump and a turbo molecular pump and heating at 473 K for 12 hours at 1 kPa or less).

According to the adsorption isotherm, the metal-organic framework [$Cu_4I_4$Lp] did not capture nitrogen gas, but a specific capture of $CO_2$ was observed. Hysteresis is also observed in $CO_2$ capture and desorption.

It is generally understood that since the pore is an isolated void that is connected to the outside only through a passage through which a sphere having a diameter equivalent to the kinetic diameter of gaseous helium (2.57 Å) cannot pass, nitrogen molecules having a kinetic diameter larger than that of gaseous helium cannot be captured. On the other hand, the fact that $CO_2$ was captured and desorbed suggests that the three-dimensional structure of the metal-organic framework [$Cu_4I_4$Lp] was changed by $CO_2$, creating a channel through which $CO_2$ could pass.

<Characterization After Solvent (acetonitrile) Removal>

After removing acetonitrile in the pores of the metal-organic framework [$Cu_4I_4$Lp], single crystal X-ray structural analysis and FT-IR measurements were performed. Note that the FT-IR measurement results will be explained in the section on characterization after $CO_2$ capture, which will be described later.

<<Method for Removing Solvent>>

In the adsorption isotherm using BELSORP MAX, when heated at 200°C for 12 hours or more at 1 kPa or less, the powdered metal-organic framework [$Cu_4I_4$Lp] exhibits adsorption properties that capture the gas, confirming the removal of the solvent.

Therefore, to remove the solvent in a single crystal state, one spatula of the single crystal of the metal-organic framework [$Cu_4I_4$Lp] was placed in a 10 mL ampoule tube, and the inside of the ampoule tube was evacuated for 1 hour using a rotary pump. The ampoule tube was closed under vacuum and heated in an oven at 240°C for 14 hours.

<<Single Crystal X-ray Structure Analysis>>

Measurement was performed with BL-5A of the Photon Factory (PF) of the Institute of Materials Structure Science (IMSS) of the High Energy Accelerator Research Organization (KEK). The ampoule tube was carried in a closed state, and immediately after opening the ampoule tube, it was immersed in oil. Therefore, measurement was performed in the air. Table 3 shows the analysis results in detail. Note that the results were obtained after removing the solvent (acetonitrile) in the crystals.

[Table 3]

| | [Cu4I4Lp] (without acetonitrile) |
|---|---|
| Empirical formula | $C_{34}H_{30}Cu_4I_4N_8$ |
| Formula weight | 1312.42 |
| Temperature/K | 90 |
| Crystal system | *tetraqonal* |
| Space group | $I4_1/a$ |
| a/Å | 12.3500(6) |
| b/Å | 12.3500(6) |
| c/Å | 28.851(2) |
| $\alpha$/° | 90 |
| $\beta$/° | 90 |
| $\gamma$/° | 90 |
| Volume/Å $^3$ | 4400.4(6) |
| Z | 4 |
| $\rho_{calc}$g/cm$^3$ | 0.298 |
| $\mu$/mm$^{-1}$ | 1.347 |
| F (000) | 342.0 |

(continued)

| | [Cu4I4Lp] (without acetonitrile) |
|---|---|
| Radiation | $\lambda$ = 0.750 |
| 2$\theta$ ranqe for data collection/° | 3.786 to 71.416 |
| Index ranges | -15<h<15, -18< k< 18, -43<l<36 |
| Reflections collected | 18479 |
| Independent reflections | 3407 [$R_{int}$ = 0.0436, $R_{sigma}$ = 0.0327] |
| Data/restraints/parameters | 3407/0/117 |
| Goodness-of-fit on $F^2$ | 1.178 |
| Final R indexes [I>=2$\sigma$ (I)] | $R_1$ = 0.0468, $wR_2$ = 0.1482 |
| Final R indexes [all data] | $R_1$ = 0.0551, $wR_2$=0.1743 |
| Largest diff. peak/hole / e Å$^{-3}$ | 1.00/-1.69 |

FIG. 12 shows the results of crystal X-ray structure analysis of the metal-organic framework [$Cu_4I_4$Lp] without acetonitrile.

Single crystal X-ray structural analysis confirmed that the single crystal, after vacuum heating, retained almost no solvent. Furthermore, even after the solvent was removed, the point group of the crystal structure did not change, and the lattice size showed almost no change, indicating that the structure was maintained.

<Characterization after $CO_2$ Capture>

After adsorbing $CO_2$ to the metal-organic framework [$Cu_4I_4$Lp], single crystal X-ray structure analysis and FT-IR measurement were performed.

<<$CO_2$ Capture Method>>

BELSORP MAX was used for $CO_2$ capture. Two spatulas of the single crystal, from which the solvent had been removed by vacuum heating, were added to the BELSORP measurement container. Heating was performed at 200°C for 12 hours at 1 kPa or less by the same operation as in the adsorption isotherm measurement. After heating, the inside of the container was vacuumed and purged with $CO_2$. The inside of the container was left filled with $CO_2$ for one day. The single crystal was taken out from the container and transferred to a vial (5 mL). The crystals were stored under a high concentration of $CO_2$ by spraying $CO_2$ into the vial using a commercially available $CO_2$ spray and immediately closing the cap.

<<Single Crystal X-ray Structure Analysis>>

Measurement was performed with BL-5A of the Photon Factory (PF) of the Institute of Materials Structure Science (IMSS) of the High Energy Accelerator Research Organization (KEK). After the single crystal was transferred from the BELSORP measurement container to a vial, measurement was performed one day later. At the time of measurement, the single crystal was removed from the container and immediately immersed in oil. Table 4 shows the analysis results in detail. Note that the results were obtained after $CO_2$ capture.

[Table 4]

| | [Cu4I4Lp] (with $CO_2$ adsorption) |
|---|---|
| Empirical formula | $C_{35}H_{30}Cu_4I_4N_8O_2$ |
| Formula weight | 1356.43 |
| Temperature/K | 90 |
| Crystal system | *tetragonal* |

(continued)

| | [Cu4I4Lp] (with $CO_2$ adsorption) |
|---|---|
| Space group | *I4₁/a* |
| a/Å | 12.362(1) |
| b/Å | 12.362(1) |
| c/Å | 28.780(6) |
| α/° | 90 |
| β/° | 90 |
| γ/° | 90 |
| Volume/Å$^3$ | 4398(1) |
| Z | 1 |
| $\rho_{calc}$g/cm$^3$ | 0.304 |
| μ/mm$^{-1}$ | 1.349 |
| F (000) | 350.0 |
| Radiation | (λ = 0.750) |
| 2θ range for data collection/° | 3.784 to 71.352 |
| Index ranges | -19<h<18, -19<k<19, -36<l<43 |
| Reflections collected | 16910 |
| Independent reflections | 3351 [$R_{int}$ = 0.0390, $R_{sigma}$ = 0.0318] |
| Data restraints parameters | 3351/0/131 |
| Goodness-of-fit on F$^2$ | 1.166 |
| Final R indexes [I>=2σ (I)] | $R_1$ =00583, w$R_2$ = 0.1633 |
| Final R indexes [all data] | $R_1$ =00631, w$R_2$ = 0.1685 |
| Largest diff. peak/hole / e Å$^{-3}$ | 0.76/-1.31 |

FIG. 13 shows the results of single crystal X-ray structure analysis.

A linear electron density distribution, which had not been observed after the solvent was removed, was observed in the areas corresponding to the pores. It can be applied as $CO_2$, suggesting that $CO_2$ was captured within the pores. Since this measurement was performed in the air, this structure is believed to capture $CO_2$ even when it does not exist in a $CO_2$ atmosphere.

<<FT-IR Measurement>>

The FT-IR spectrum of the metal-organic framework [$Cu_4I_4$Lp] after $CO_2$ capture is shown in FIG. 14 ([I]).

The FT-IR spectrum of the metal-organic framework [$Cu_4I_4$Lp] when there was no $CO_2$ capture without acetonitrile is also shown in FIG. 14 ([II]). This FT-IR spectrum was obtained by powdering the single crystal of the metal-organic framework [$Cu_4I_4$Lp] in a mortar after vacuum heating, diluting it with KBr, and measuring it in the air at room temperature.

Furthermore, the FT-IR spectrum shown in FIG. 6 before the removal of the solvent (acetonitrile) is also shown in FIG. 14 ([III]).

[I]: After $CO_2$ capture
[II]: After vacuum heating
[III]: Before removal of solvent (acetonitrile)

It was confirmed that the peak [A] at a wave number of from 2200 $cm^{-1}$ to 2300 $cm^{-1}$, which was present before the solvent (acetonitrile) was removed ([III]), disappeared after vacuum heating ([II]). This peak is thought to be derived from acetonitrile as the solvent, suggesting that the solvent in the pores was entirely removed by vacuum heating. This fact shows consistency with the results obtained by single-crystal X-ray crystal structure analysis.

[A]: C=N vibration (acetonitrile)
[B]: C=O($CO_2$) asymmetric vibration

Furthermore, after $CO_2$ capture, a new peak [B] appeared from 2300 $cm^{-1}$ to 2400 $cm^{-1}$ (see [I]). This is thought to be derived from $CO_2$, and it was shown that this framework captures $CO_2$ and that it also captures $CO_2$ in the air. Furthermore, when this single crystal was left in the air for one week, it was observed that the $CO_2$ peak remained, although its height decreased. This suggests that the framework continues to capture $CO_2$ in the air.

## Claims

1. A carbon dioxide adsorbent comprising a metal-organic framework, wherein

   the metal-organic framework can capture and desorb carbon dioxide,
   isolated voids are formed inside the metal-organic framework by the metal-organic framework's three-dimensional structure,
   the isolated voids are the space that can capture carbon dioxide and does not have a channel through which carbon dioxide can pass in an ordinary state, and
   although the three-dimensional structure of the framework changes during the process where carbon dioxide is captured within isolated voids and the process where carbon dioxide is released from isolated voids, the three-dimensional structure of the metal-organic framework when carbon dioxide is captured within the isolated voids is the same as when carbon dioxide is not captured within the isolated voids.

2. The carbon dioxide adsorbent according to claim 1, wherein the metal-organic framework has a BET-specific surface area of 1 $m^2/g$ or less in a specific surface area measurement using $N_2$.

3. The carbon dioxide adsorbent according to claim 1 or 2, wherein the metal-organic framework forms an interpenetrating structure in which two frameworks interpenetrate each other.

4. The carbon dioxide adsorbent according to claim 3, wherein an element constituting the isolated voids and carbon dioxide captured within the isolated voids do not form a chemical bond in the metal-organic framework.

5. The carbon dioxide adsorbent according to claim 4, wherein the metal-organic framework comprises at least one of groups II to XIV elements as a constituent element.

6. The carbon dioxide adsorbent according to claim 4, wherein the metal-organic framework comprises a halogen element as a constituent element.

7. The carbon dioxide adsorbent according to claim 4, wherein the metal-organic framework comprises a compound having a nitrogen-containing aromatic heterocycle as a ligand.

8. The carbon dioxide adsorbent according to claim 7, wherein the compound having a nitrogen-containing aromatic heterocycle is a compound represented by the following Formula (1):

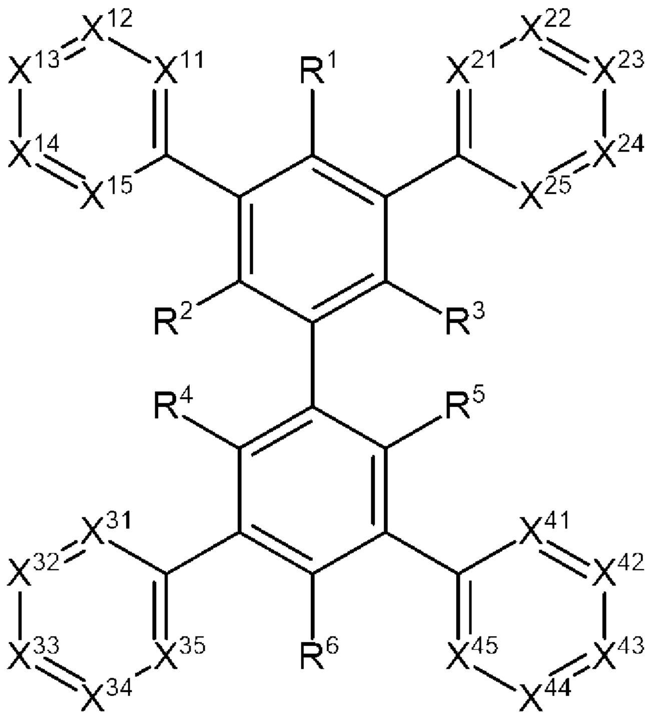

(1)

(in Formula (1),

$X^{11}$ and $X^{13}$ are N, and $X^{12}$, $X^{14}$, and $X^{15}$ are CR, or $X^{12}$ and $X^{14}$ are N, and $X^{11}$, $X^{13}$, and $X^{15}$ are CR, or $X^{11}$ and $X^{15}$ are N, and $X^{12}$, $X^{13}$, and $X^{14}$ are CR;
$X^{21}$ and $X^{23}$ are N, and $X^{22}$, $X^{24}$, and $X^{25}$ are CR, or $X^{22}$ and $X^{24}$ are N, and $X^{21}$, $X^{23}$, and $X^{25}$ are CR, or $X^{21}$ and $X^{25}$ are N, and $X^{22}$, $X^{23}$, and $X^{24}$ are CR;
$X^{31}$ and $X^{33}$ are N, and $X^{32}$, $X^{34}$, and $X^{35}$ are CR, or $X^{32}$ and $X^{34}$ are N, and $X^{31}$, $X^{33}$, and $X^{35}$ are CR, or $X^{31}$ and $X^{35}$ are N, and $X^{32}$, $X^{33}$, and $X^{34}$ are CR;
$X^{41}$ and $X^{43}$ are N, and $X^{42}$, $X^{44}$, and $X^{45}$ are CR, or $X^{42}$ and $X^{44}$ are N, and $X^{41}$, $X^{43}$, and $X^{45}$ are CR, or $X^{41}$ and $X^{45}$ are N, and $X^{42}$, $X^{43}$, and $X^{44}$ are CR;
each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and
$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

**9.** The carbon dioxide adsorbent according to claim 8, wherein the compound represented by Formula (1) is a compound represented by the following Formula (1-1):

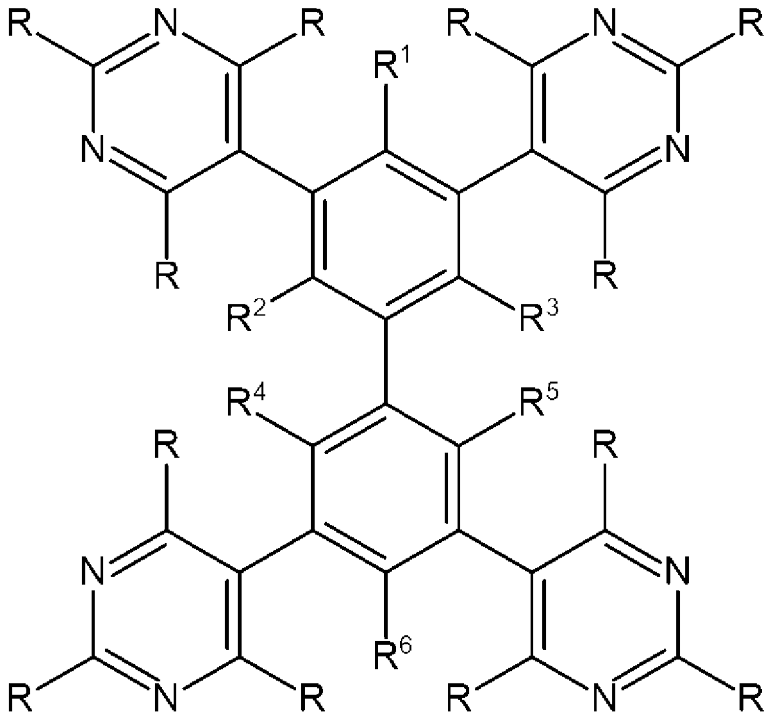

(1-1)

(in Formula (1-1),

each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and

$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

**10.** The carbon dioxide adsorbent according to claim 4, wherein the metal-organic framework has a composition formula, which is represented by $Cu_4I_4L$, with a ligand being L.

**11.** A metal-organic framework comprising at least one of groups II to XIV elements as a constituent element and a compound represented by the following Formula (1) as a ligand:

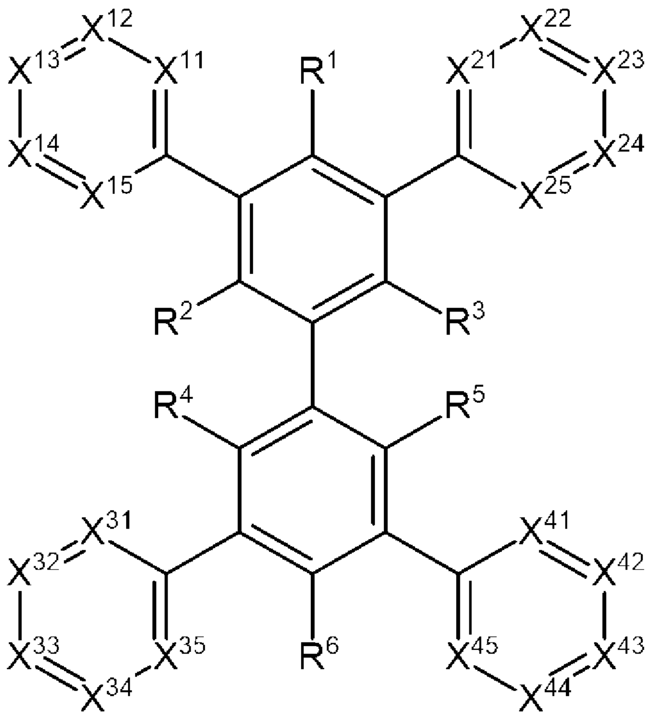

(1)

(in Formula (1),

$X^{11}$ and $X^{13}$ are N, and $X^{12}$, $X^{14}$, and $X^{15}$ are CR, or $X^{12}$ and $X^{14}$ are N, and $X^{11}$, $X^{13}$, and $X^{15}$ are CR, or $X^{11}$ and $X^{15}$ are N, and $X^{12}$, $X^{13}$, and $X^{14}$ are CR;
$X^{21}$ and $X^{23}$ are N, and $X^{22}$, $X^{24}$, and $X^{25}$ are CR, or $X^{22}$ and $X^{24}$ are N, and $X^{21}$, $X^{23}$, and $X^{25}$ are CR, or $X^{21}$ and $X^{25}$ are N, and $X^{22}$, $X^{23}$, and $X^{24}$ are CR;
$X^{31}$ and $X^{33}$ are N, and $X^{32}$, $X^{34}$, and $X^{35}$ are CR, or $X^{32}$ and $X^{34}$ are N, and $X^{31}$, $X^{33}$, and $X^{35}$ are CR, or $X^{31}$ and $X^{35}$ are N, and $X^{32}$, $X^{33}$, and $X^{34}$ are CR;
$X^{41}$ and $X^{43}$ are N, and $X^{42}$, $X^{44}$, and $X^{45}$ are CR, or $X^{42}$ and $X^{44}$ are N, and $X^{41}$, $X^{43}$, and $X^{45}$ are CR, or $X^{41}$ and $X^{45}$ are N, and $X^{42}$, $X^{43}$, and $X^{44}$ are CR;
each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and
$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

**12.** The metal-organic framework according to claim 11, wherein the compound represented by Formula (1) is a compound represented by the following Formula (1-1):

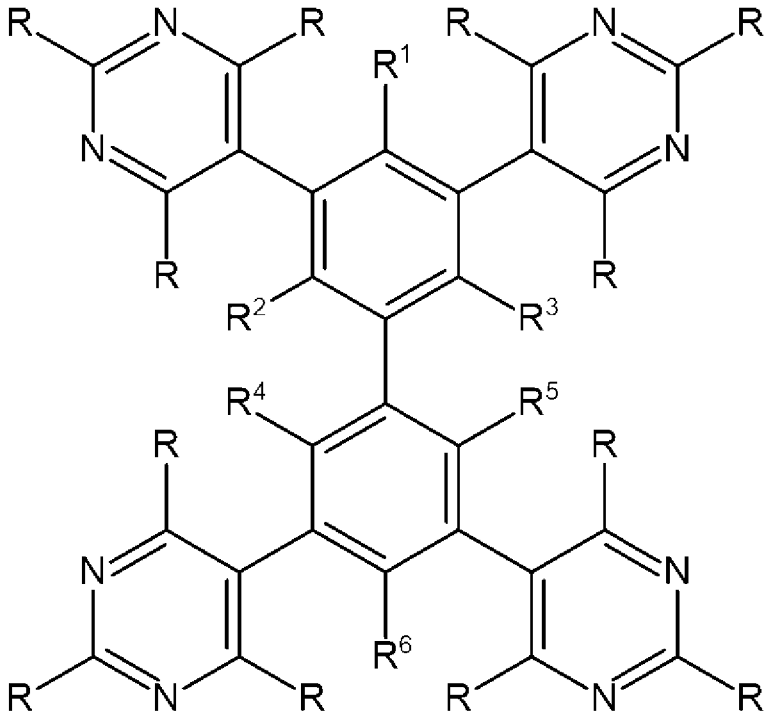

(1-1)

(in Formula (1-1),

each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and

$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

**13.** The metal-organic framework according to claim 11, which comprises a halogen element as a constituent element.

**14.** The metal-organic framework according to claim 11, which has a composition formula represented by $Cu_4I_4L$, with the ligand being L.

**15.** The metal-organic framework according to claim 11, wherein the constituent element contains copper, and the ligand is coordinated to the copper by one nitrogen atom of two nitrogen atoms of a pyrimidine ring of the ligand.

**16.** The metal-organic framework according to claim 11, wherein the constituent element contains copper and iodine, and $Cu_4I_4$ is present in a cubane-type structure in the metal-organic framework.

**17.** The metal-organic framework according to claim 11, wherein the constituent element contains copper and iodine,

$Cu_4I_4$ is present in a cubane-type structure in the metal-organic framework,

the metal-organic framework has a composition formula, which is represented by $Cu_4I_4L$, with the ligand being L, and

the ligand is coordinated to the copper by one nitrogen atom of two nitrogen atoms of a pyrimidine ring of the ligand.

**18.** A carbon dioxide adsorbent comprising the metal-organic framework according to any one of claims 11 to 17.

**19.** A compound represented by the following Formula (1):

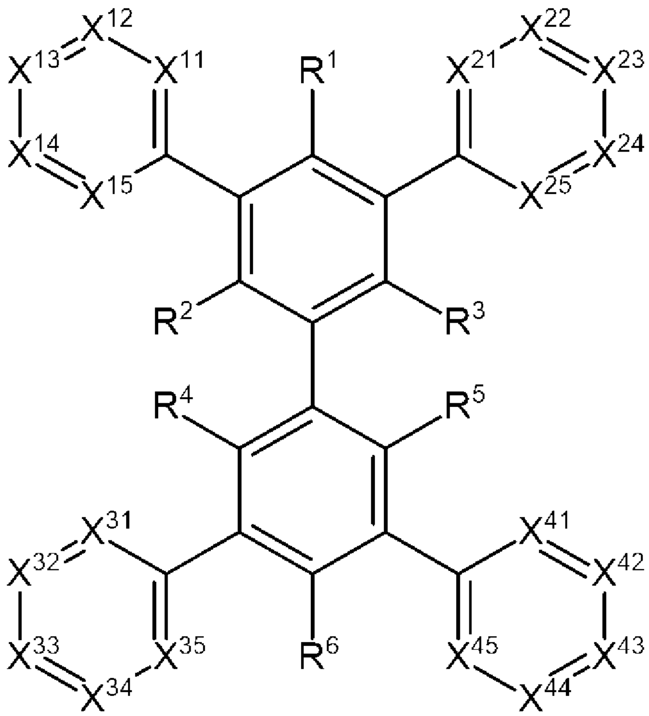

(1)

(in Formula (1),

$X^{11}$ and $X^{13}$ are N, and $X^{12}$, $X^{14}$, and $X^{15}$ are CR, or $X^{12}$ and $X^{14}$ are N, and $X^{11}$, $X^{13}$, and $X^{15}$ are CR, or $X^{11}$ and $X^{15}$ are N, and $X^{12}$, $X^{13}$, and $X^{14}$ are CR;
$X^{21}$ and $X^{23}$ are N, and $X^{22}$, $X^{24}$, and $X^{25}$ are CR, or $X^{22}$ and $X^{24}$ are N, and $X^{21}$, $X^{23}$, and $X^{25}$ are CR, or $X^{21}$ and $X^{25}$ are N, and $X^{22}$, $X^{23}$, and $X^{24}$ are CR;
$X^{31}$ and $X^{33}$ are N, and $X^{32}$, $X^{34}$, and $X^{35}$ are CR, or $X^{32}$ and $X^{34}$ are N, and $X^{31}$, $X^{33}$, and $X^{35}$ are CR, or $X^{31}$ and $X^{35}$ are N, and $X^{32}$, $X^{33}$, and $X^{34}$ are CR;
$X^{41}$ and $X^{43}$ are N, and $X^{42}$, $X^{44}$, and $X^{45}$ are CR, or $X^{42}$ and $X^{44}$ are N, and $X^{41}$, $X^{43}$, and $X^{45}$ are CR, or $X^{41}$ and $X^{45}$ are N, and $X^{42}$, $X^{43}$, and $X^{44}$ are CR;
each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and
$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

**20.** The compound according to claim 19, which is a compound represented by the following Formula (1-1):

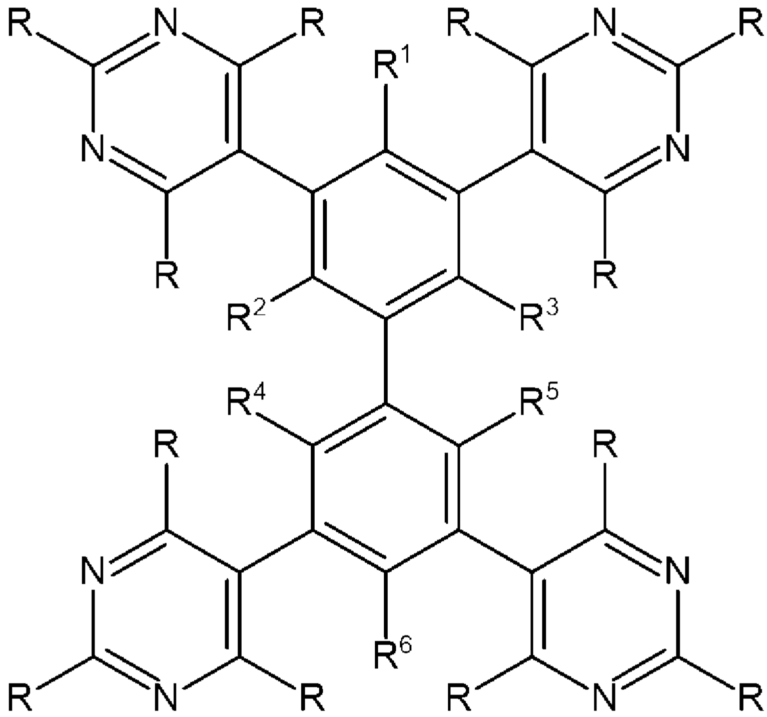

(1-1)

(in Formula (1-1),

each R is independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom); and

$R^1$ to $R^6$ are each independently a hydrogen atom, a halogen atom, an alkyl group optionally substituted with a halogen atom, an alkyloxy group optionally substituted with a halogen atom, or an aryl group (the aryl group may be substituted with a halogen atom, an alkyl group optionally substituted with a halogen atom, or an alkyloxy group optionally substituted with a halogen atom).

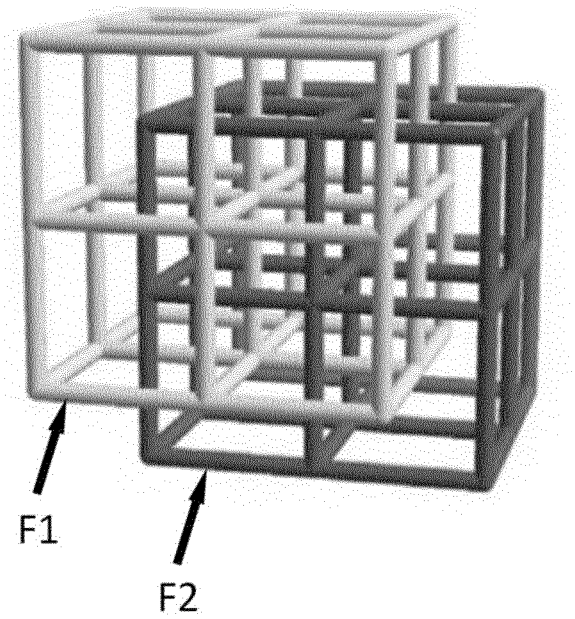


Fig. 1

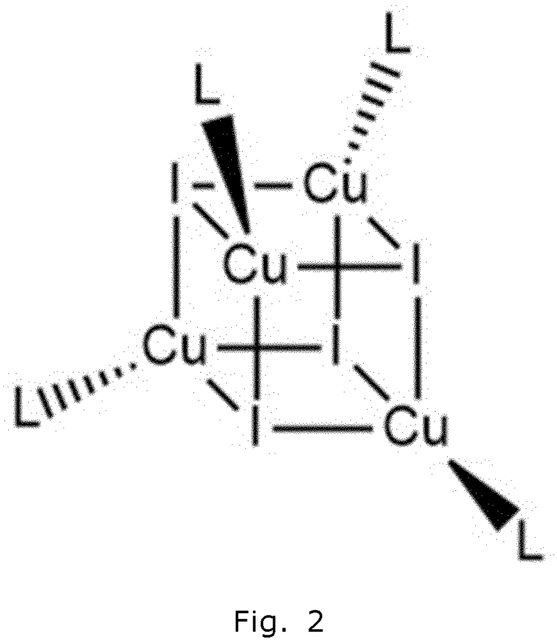

Fig. 2

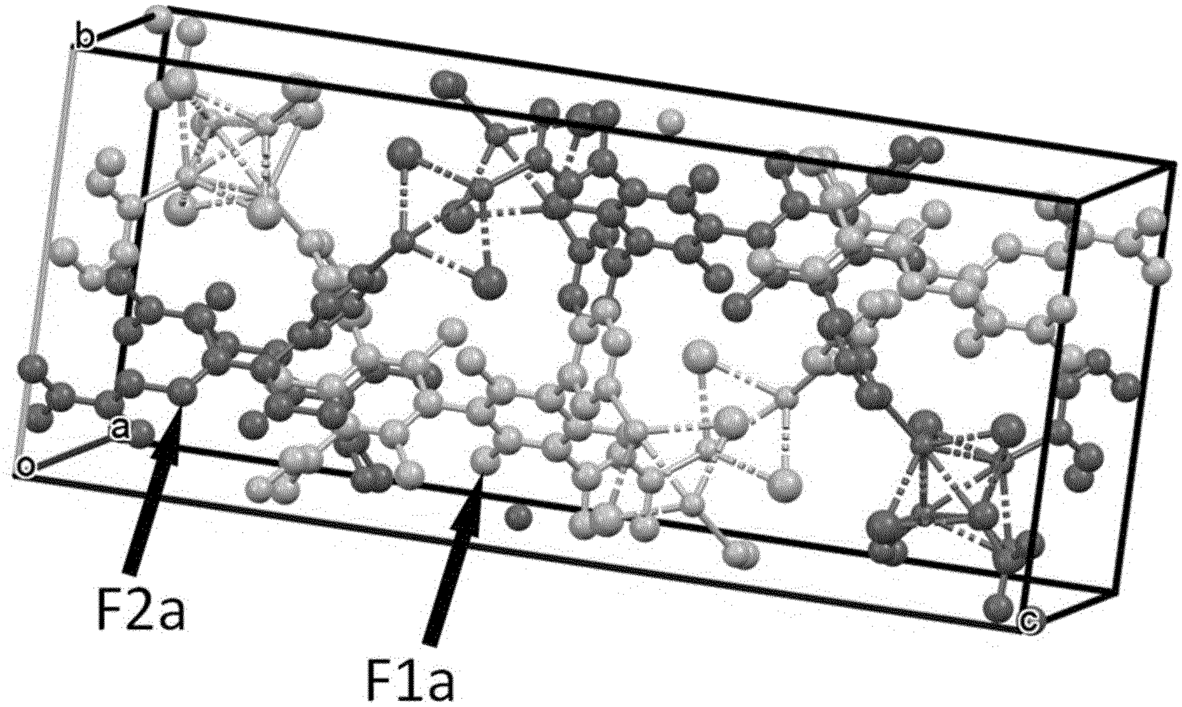


Fig. 3

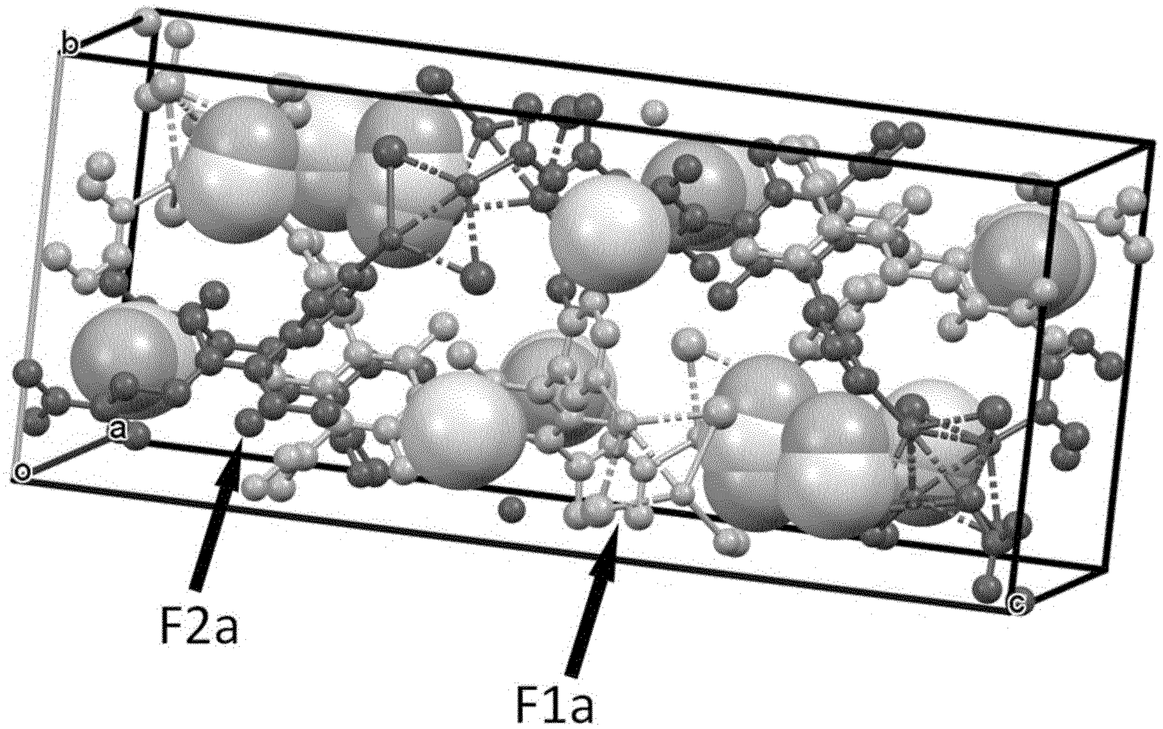


Fig. 4

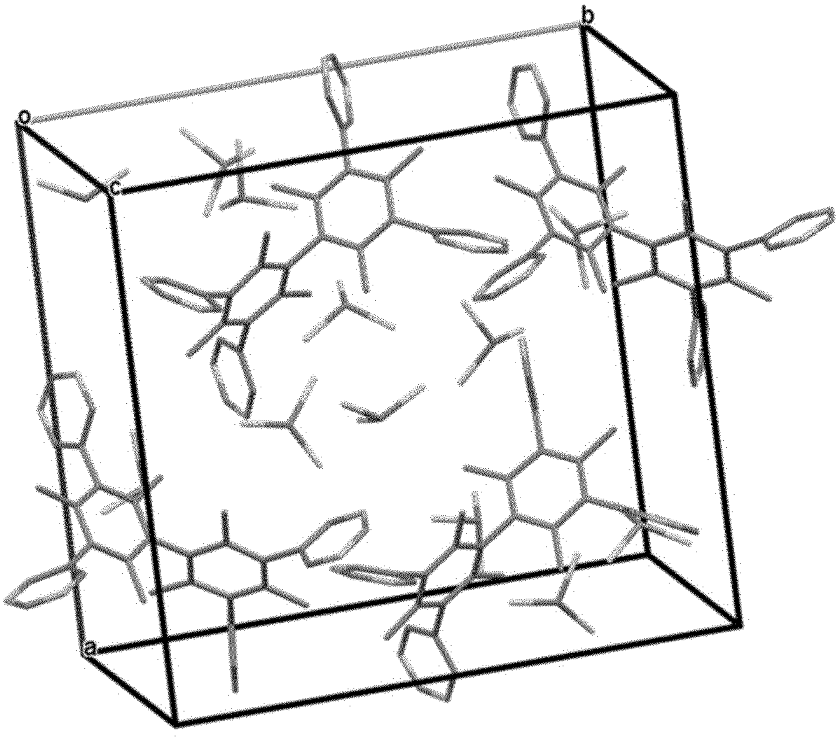

Fig. 5A

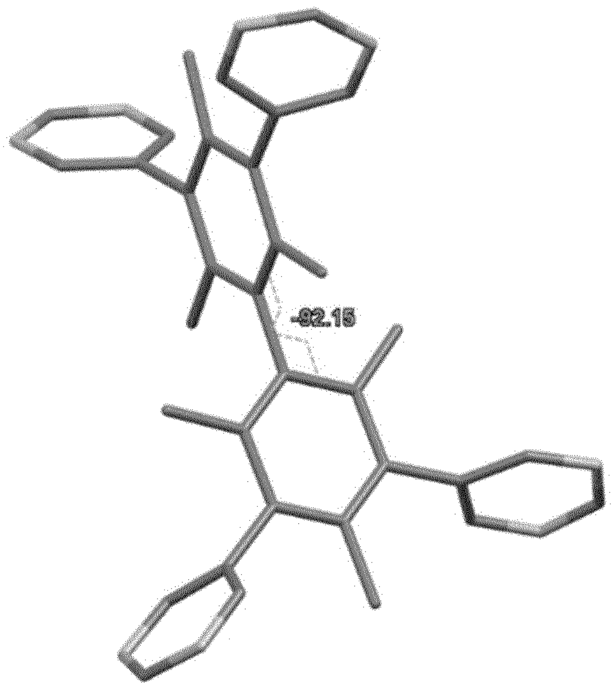

Fig. 5B

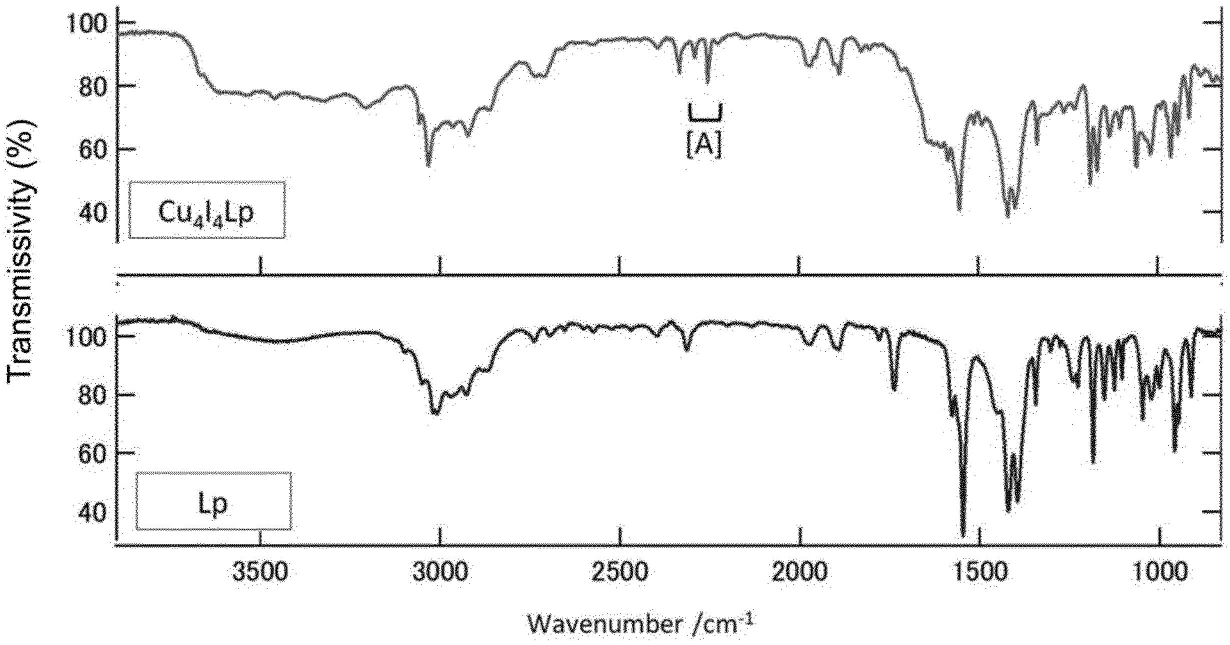
Transmissivity (%)
100
80
60
40
Cu4I4Lp
[A]
Lp
3500
3000
2500
2000
1500
1000
Wavenumber /cm-1

Fig. 6

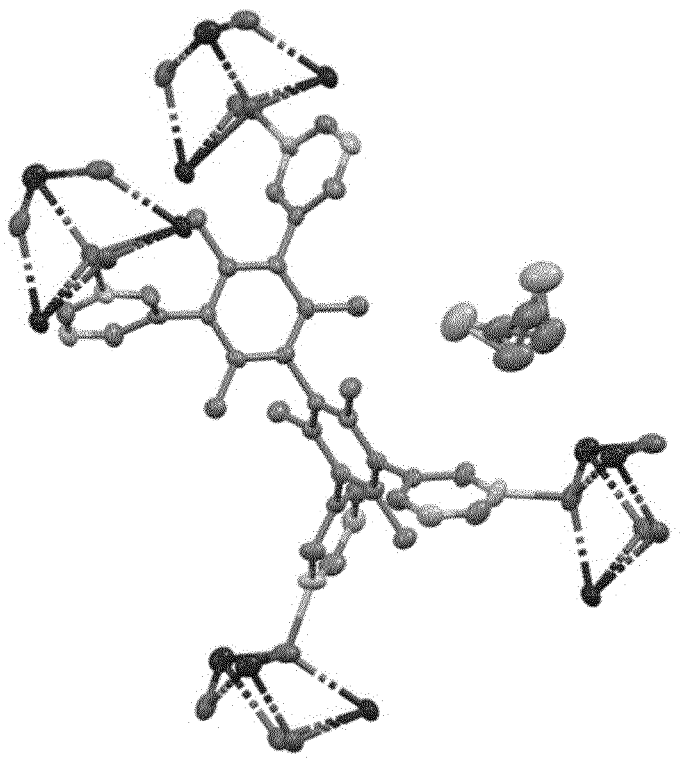

Fig. 7A

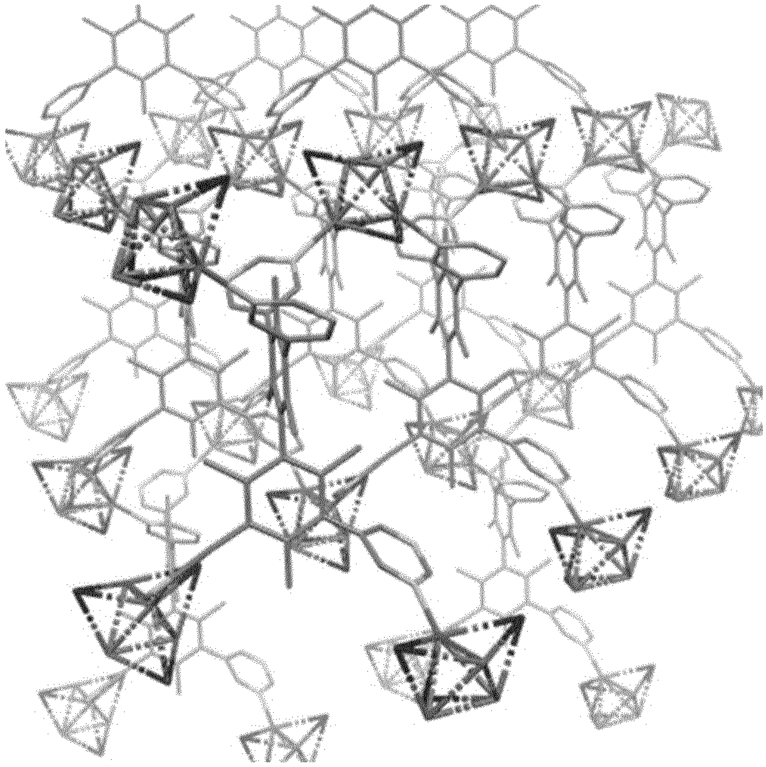

Fig. 7B

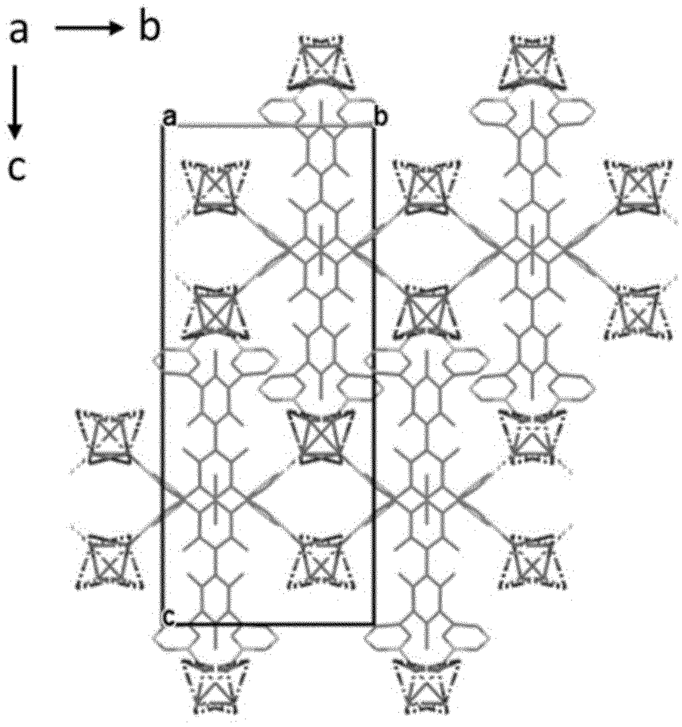


Fig. 8A

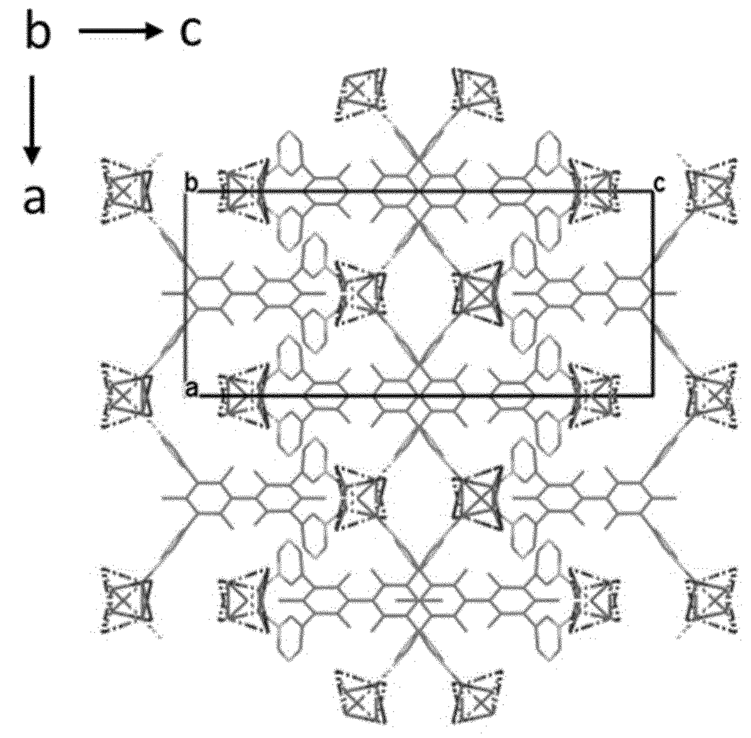


Fig. 8B

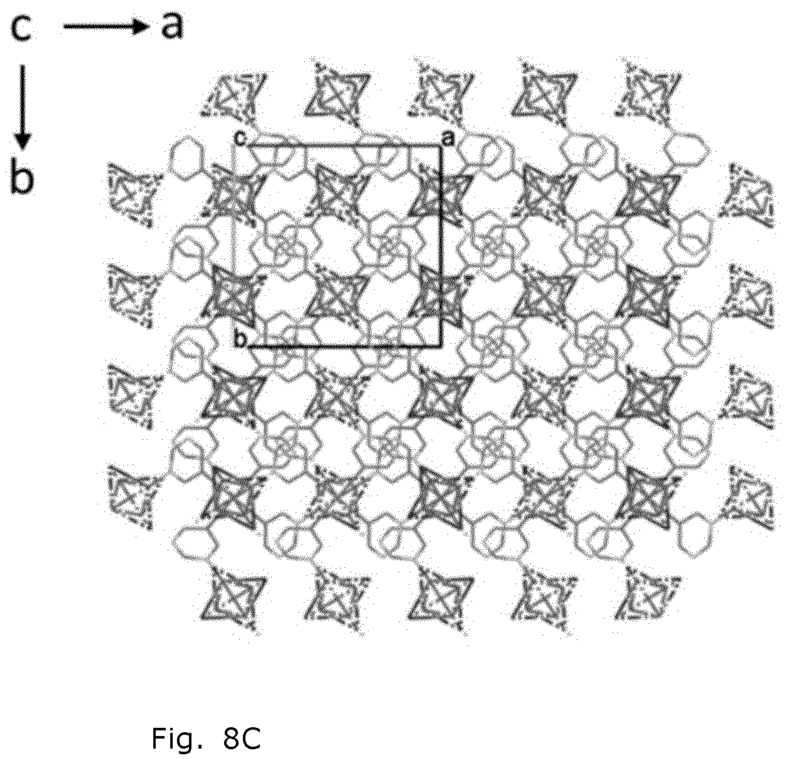


Fig. 8C

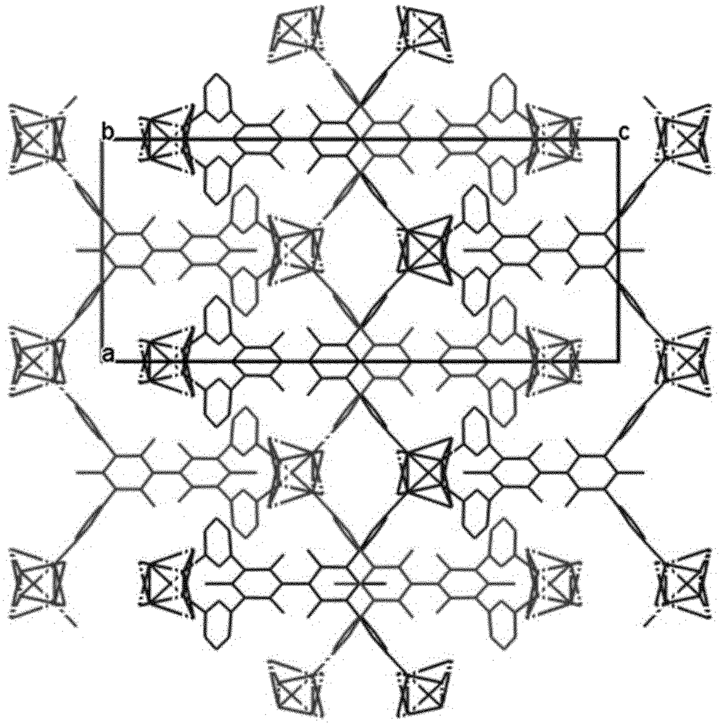


Fig. 9A

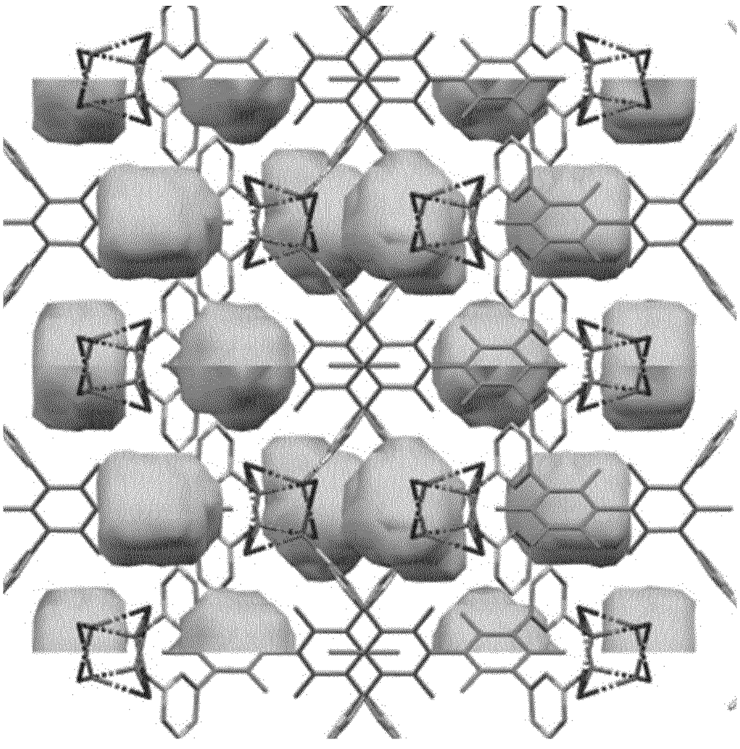

Fig. 9B

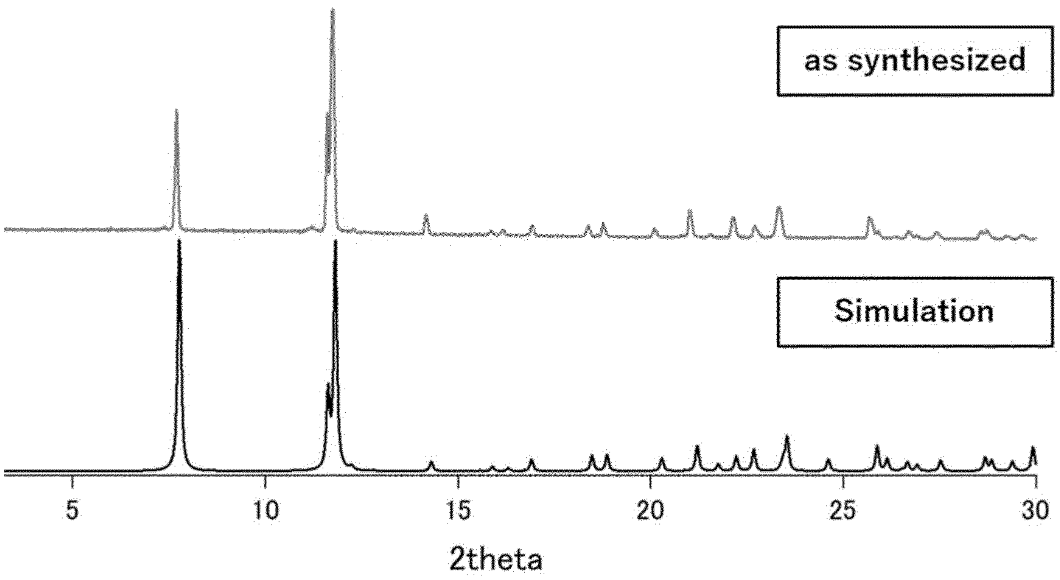
as synthesized
Simulation
5
10
15
20
25
30
2theta

Fig. 10

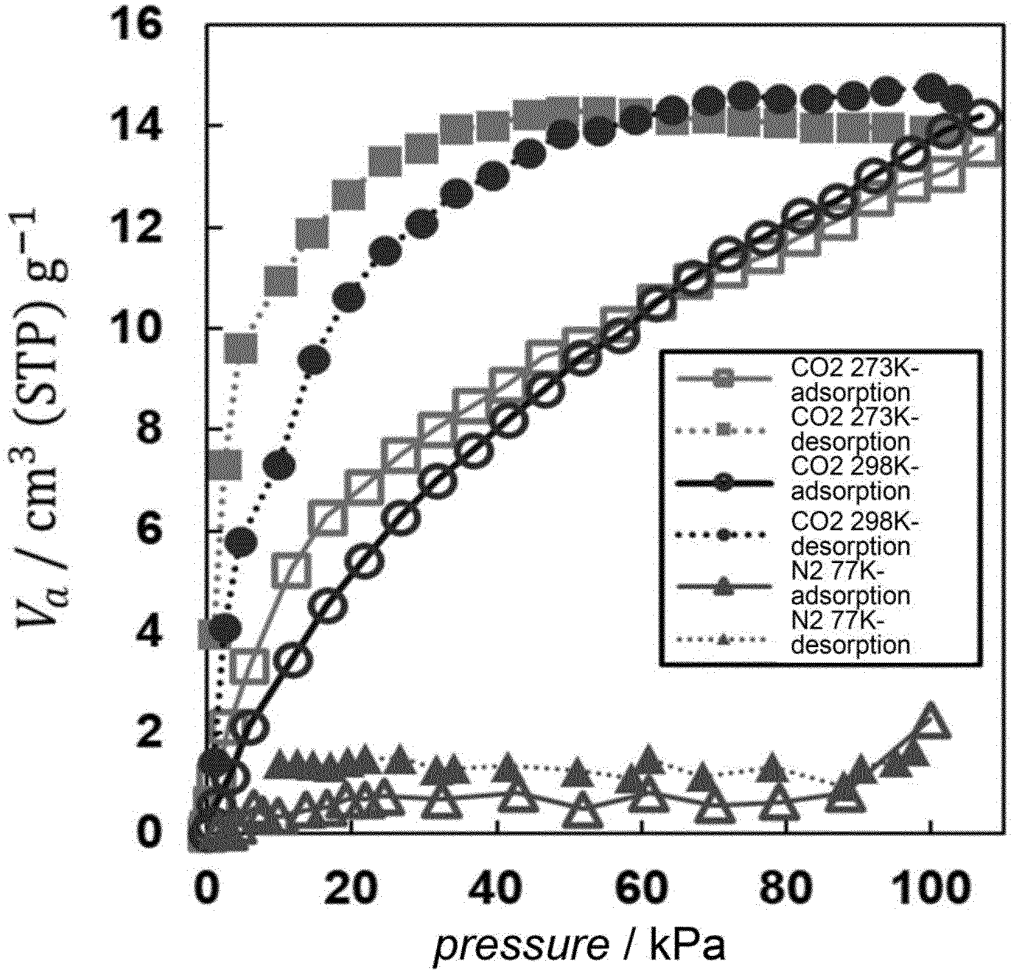
16
14
12
10
8
6
4
2
0
$V_a$ / $cm^3$ (STP) $g^{-1}$
0
20
40
60
80
100
pressure / kPa
CO2 273K-adsorption
CO2 273K-desorption
CO2 298K-adsorption
CO2 298K-desorption
N2 77K-adsorption
N2 77K-desorption

Fig. 11

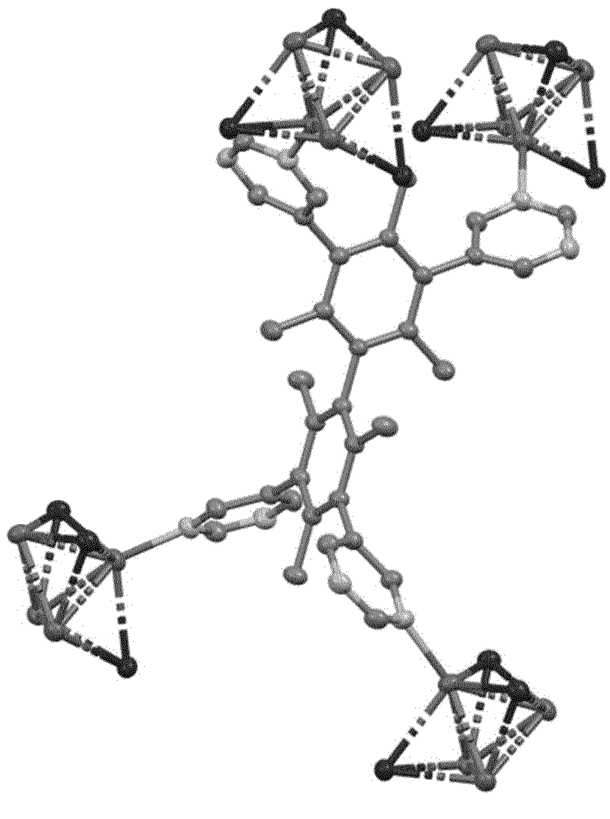

Fig. 12

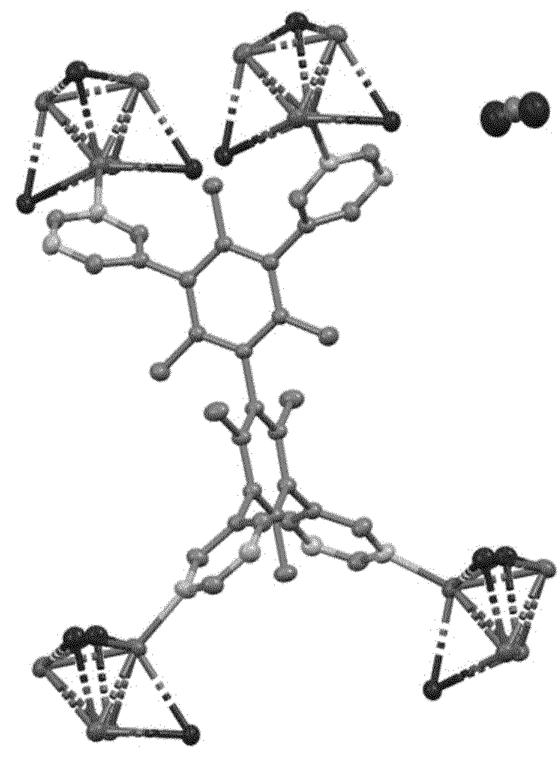

Fig. 13

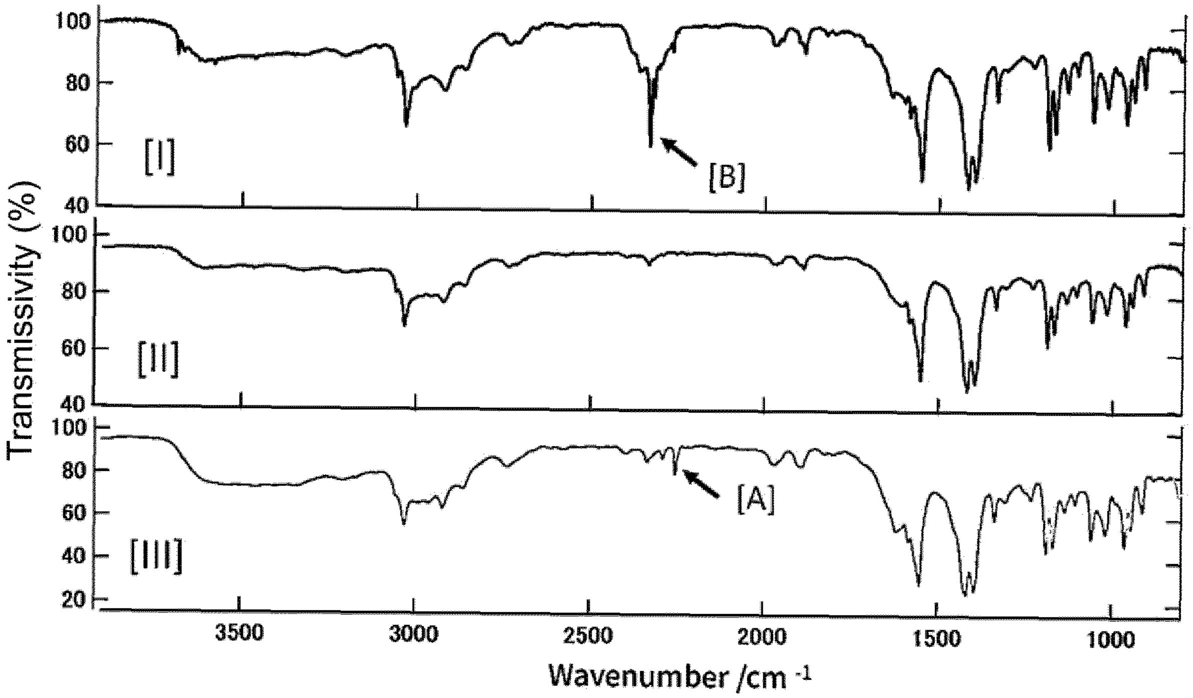
100
80
60
40
100
80
60
40
100
80
60
40
20
Transmissivity (%)
[I]
[B]
[II]
[III]
[A]
3500
3000
2500
2000
1500
1000
Wavenumber /cm -1

Fig. 14

## INTERNATIONAL SEARCH REPORT

International application No.
**PCT/JP2022/032745**

**A. CLASSIFICATION OF SUBJECT MATTER**

***B01J 20/22***(2006.01)i; ***C07D 239/26***(2006.01)i; ***C07F 1/00***(2006.01)i
FI: B01J20/22 A; C07D239/26; C07F1/00 C CSP

According to International Patent Classification (IPC) or to both national classification and IPC

**B. FIELDS SEARCHED**

Minimum documentation searched (classification system followed by classification symbols)

B01J20/22; C07D239/26; C07F1/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | Inorg. Chem. 06 July 2015, vol. 54, pp. 6829-6835<br>entire text | 1-20 |
| A | JP 2012-20956 A (KURARAY CO., LTD.) 02 February 2012 (2012-02-02)<br>entire text | 1-20 |
| A | JP 2013-40147 A (KURARAY CO., LTD.) 28 February 2013 (2013-02-28)<br>entire text | 1-20 |
| A | JP 2016-196417 A (NIPPON STEEL & SUMITOMO METAL CORP.) 24 November 2016 (2016-11-24)<br>entire text | 1-20 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2022** | **11 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/032745**

| Patent document cited in search report | Publication date (day/month/year) | Patent family member(s) | Publication date (day/month/year) |
|---|---|---|---|
| JP 2012-20956 A | 02 February 2012 | (Family: none) | |
| JP 2013-40147 A | 28 February 2013 | (Family: none) | |
| JP 2016-196417 A | 24 November 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZHONG LI et al.** *ACS Sustainable Chem. Eng.,* 2020, vol. 8 (41), 15378-15404
- **CALOGERO GIANCARLO PISCOPO et al.** *ChemPlusChem,* 2020, vol. 85, 538-547
- *Isotope News,* August 2017, vol. 752, 12-15
- **CHRISTIAN S. DIERCKS ; OMAR M. YAGHI.** *Science,* 2017, vol. 355 (6328), eaal158
- **SHI-BIN REN et al.** *CrystEngComm,* 2009, vol. 11, 1834-1836